# EUROPÄISCHE PATENTSCHRIFT

(11) **EP 0 557 879 B1**
(45) Veröffentlichungstag und Bekanntmachung des Hinweises auf die Patenterteilung: **08.01.1997**
(21) Anmeldenummer: 93102453.3
(22) Anmeldetag: 17.02.1993
(51) Int. Cl.: C07D 403/04, C07D 233/38, A61K 31/505

(54) **4-Amino-2-ureido-pyrimidin-5-carbonsäureamide, Verfahren zu deren Herstellung, diese Verbindungen enthaltende Arzneimittel und deren Verwendung**
4-amino-2-ureido-5-pyrimidincarboxamide, process for their preparation, medicines containing those compounds and their use
4-amino-2-uréido-5-pyrimidine carboxamide, procédé pour leur préparation, médicaments contenant ces composés et leur utilisation

(30) Priorität: 22.02.1992 DE 4205484
(43) Veröffentlichungstag der Anmeldung: 01.09.1993
(73) Patentinhaber: HOECHST AKTIENGESELLSCHAFT, 65926 Frankfurt am Main (DE)
(72) Erfinder: Kampe, Klaus-Dieter, Dr., W-6232 Bad Soden/Ts. (DE); Granzer, Ernold, Dr. Dr., W-6233 Kelkheim/Ts. (DE); Leineweber, Michael, Dr., W-6230 Frankfurt/M. (DE); Rackup, Gerhard, Dr., W-6270 Idstein/Ts. (DE); Böger, Hans Georg, Dr., W-6238 Hofheim/Ts. (DE)

(56) Entgegenhaltungen:
- EP-A- 0 012 361
- EP-A- 0 206 297

## Beschreibung

Die Erfindung betrifft tertiäre Amide der 4-Amino-2-ureido-pyrimidin-5-carbonsäure sowie deren Säureadditionssalze.
Insbesondere betrifft die Erfindung substituierte 4-Amino-2-(2-oxo-imidazolidin-1-yl)-pyrimidin-5-carbonsäure-[N-(cyclo)alkyl-N-(substituierte)phenyl-amide] und deren Säureadditionssalze.

Es ist bereits beschrieben worden, 4-Amino-2-ureido-pyrimidin-5-carbonsäure-(N-phenyl-amide) zur Behandlung von Adipositas und von Lipidstoffwechselstörungen [vgl. Europ. Patentschrift 0012361 = US-Patent Nr. 4285946] zu verwenden. Außerdem ist beschrieben worden, eine bezüglich ihrer Struktur spezielle Gruppe dieser Verbindungen, nämlich entsprechende N-(3-Trifluormethyl-phenyl)-amide, zur Prophylaxe gegen und Behandlung von Thrombosen anzuwenden [vgl. Europ. Patentschrift 0206297 = US-Patent Nr. 4705792].
Die Verträglichkeit der als Arzneimittel vorgeschlagenen N-Phenylamide, und zwar der monosubstituierten, sekundären Amide ist jedoch nicht ganz befriedigend. Diese sekundären Amide hemmen bei zu hoher Dosierung in einem gewissen Grad die Proliferation einiger Zellarten. Eine solche Nebenwirkung ist bei einer Verwendung als Therapeutika zur Behandlung von Lipidstoffwechselstörungen in dieser Form unerwünscht.

Der Erfindung lag die Aufgabe zugrunde, Verbindungen zur Verfügung zu stellen, die bei guter Verträglichkeit eine therapeutisch verwertbare hypolipidämische Wirkung entfalten. In diesem Zusammenhang bestand die Aufgabe insbesondere auch darin, Verbindungen zu finden, bei denen neben ausreichender hypolipidämischer Wirksamkeit die antiproliferativen Eigenschaften im Vergleich zu den in der Europäischen Patentschrift 0012361 beschriebenen Verbindungen nur noch in einem stark reduzierten Maß oder garnicht mehr zu beobachten sind.

Als eine weitere unvorteilhafte Eigenschaft der bereits als Arzneimittel vorgeschlagenen, in der Europäischen Patentschrift 0012361 beschriebenen sekundären N-Phenylamide ist deren Schwerlöslichkeit - sowohl als Base wie als Säureadditionssalz - in Wasser sowie in physiologisch tolerablen Lösemitteln bzw. Lösemittelmischungen, inkl. Mischungen von solchen Lösemitteln mit Wasser, anzusehen.

Es wurde nun gefunden, daß tertiäre 4-Amino-2-ureido-pyrimidin-5-carbonsäure-(N-phenyl-amide), deren Amidstickstoffatom zweifach substituiert ist, also neben dem substituierten Phenylrest einen weiteren und zwar einen aliphatischen oder cycloaliphatischen Kohlenwasserstoffrest trägt, eine gute lipidsenkende Wirkung entfalten, wobei die das Wachstum bestimmter Zellen hemmende Komponente im Vergleich zu den am Amidstickstoff monosubstituierten Verbindungen kaum mehr oder nur noch schwach zu beobachten ist.

Die Erfindung betrifft daher tertiäre 4-Amino-2-ureido-pyrimidin-5-carbonsäure-amide der Formel I, 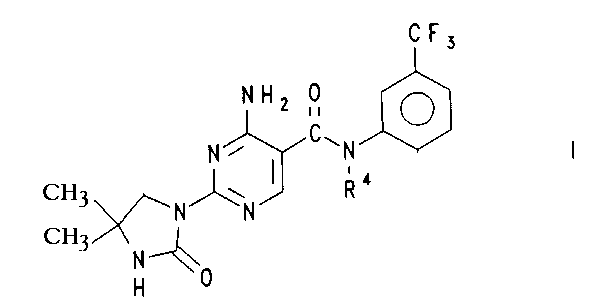 in der
R⁴ (C₂-C₃)-Alkyl, Allyl oder Cyclopropylmethyl
bedeutet sowie deren physiologisch verträgliche Säureadditionssalze.

In den vorstehenden und folgenden Ausführungen wird unter dem Ausdruck "Alkyl" jewei!s ein unverzweigter oder verzweigter Alkyl-Rest verstanden.

Für die neuen Verbindungen l, bei denen R⁴ (C₂-C₃)-Alkyl bedeutet und die in den Europäischen Patenten 0012361 und 0206297 beansprucht aber in diesen Schriften nicht beschrieben werden, wird der Charakter einer Auswahlerfindung geltend gemacht.

Es wurde ferner gefunden, daß tertiäre 4-Amino-2-ureido-pyrimidin-5-carbonsäureamide der Formel I, in der R⁴ die oben angegebene Bedeutung hat, überraschenderweise in Form von bestimmten mit mittelstarken und starken Säuren gebildeten Salzen in Wasser gut löslich sind. Unter in Wasser gut löslichen Verbindungen (Salzen) werden in Wasser leicht lösliche, lösliche und wenig lösliche Verbindungen gemäß der Definition im "Deutschen Arzneibuch" (9.Ausgabe 1986, Amtliche Ausgabe, Deutscher Apotheker-Verlag Stuttgart), Seite 19 verstanden.

Diese sehr vorteilhafte Eigenschaft, die gute Löslichkeit in Wasser von Salzen der vorgenannten Verbindungen I, ist ausgesprochen überraschend, da sowohl Salze wie auch die freien Basen analog aufgebauter Verbindungen, die neben dem Phenylrest keinen zusätzlichen Kohlenwasserstoffrest am Amid-Stickstoffatom tragen, in Wasser ausgesprochen schwerlöslich sind. So ist beispielsweise vom Hydrochlorid des 4--Amino-2-(4,4-dimethyl-imidazolidin-2-on-1-yl)-pyrimidin-5-carbonsäure-[N-(3-trifluormethyl-phenyl)-amids] in Wasser bei 23°C nur eine 0,93x10⁻³ molare Lösung herstellbar, was bedeutet, daß sich 1 Masseteil dieser Verbindung in 2500 Teilen Wasser löst und folglich sehr schwer löslich ist. Die Verbindungen I in Form der freien Basen sind wie auch 4-Amino-2-(4.4-dimethylimidazolidin-2-on-1-yl)-pyrimidin-5- carbonsäure-[N-(3-trifluormethyl-phenyl)-amid] in Form der freien Base ebenfalls in Wasser ausgesprochen schwerlöslich.

Aufgrund dieser Eigenschaften und der allgemein bei den in der Europäischen Patentschrift 0012361 beschriebenen Verbindungen festzustellenden Schwerlöslichkeit sowohl der freien Basen wie auch der Salze in Wasser, war keinesfalls zu erwarten, daß Salze der Verbindungen I in Wasser gut löslich sind.

Auch die lt. "Deutschem Arzneibuch" als "wenig löslich" bezeichnete Kategorie bedeutet gegenüber den in der Europäischen Patentschrift 0012361 beschriebenen sekundären Amiden eine überraschend hohe Steigerung der Wasserlöslichkeit.

Die sprunghafte Verbesserung der Löslichkeit von Salzen der Verbindung I in Wasser ist auch insofern überraschend, da die strukturelle Besonderheit der Verbindungen I, nämlich der Ersatz des Wasserstoffatoms am Amid-Stickstoffatom durch einen Kohlenwasserstoffrest mit den für R⁴ angegebenen Bedeutungen, in der Einführung eines gegenüber Wasserstoff lipophileren Restes besteht. Mit diesem Austausch - Wasserstoff gegen Kohlenwasserstoff - verschwindet in der Struktur eine potentielle Wasserstoffbrückenbindung. Die Struktur der Verbindungen I sollte demzufolge weniger polar werden. Das wiederum sollte keinesfalls zu einer besseren Löslichkeit der Salze in Wasser führen. Bekanntlich zeigen sekundäre Amidgruppen (mit der Struktureinheit HN-CO) eine große Tendenz zur Ausbildung von Wasserstoffbrückenbindungen, die in der Regel zu polareren und infolge dessen tendenziell hydrophileren Stoffen führen. Bei den Verbindungen I tritt gegenüber analogen Verbindungen mit einer sekundären Amidgruppe überraschenderweise der umgekehrte Effekt ein, indem hier bei den, eine tertiäre Amidgruppe enthaltenden Verbindungen I bei der Salzbildung ausgeprägt hydrophile Stoffe entstehen.

Die gute Wasserlöslichkeit von Salzen der Verbindungen I bringt einige bedeutende Vorteile für die erfindungsgemäßen Verbindungen I bei deren Verwendung als Arzneimittel.
Die in Wasser gut löslichen Salze der Verbindungen I wirken sich aufgrund dieser Eigenschaften günstig auf die Resorption dieser Wirkstoffe durch den Organismus des zu behandelnden Individuums aus. Demzufolge ergibt sich auch eine günstige Auswirkung auf die Bioverfügbarkeit des Wirkstoffs in dem betreffenden Organismus.

Vorteile bringt die gute Wasserlöslichkeit insbesondere auch für die galenischen Zubereitungen dieser Wirkstoffe. Bekanntlich sind Wirkstoffe, die gut wasserlösliche Salze bilden, galenisch einfacher und sicherer zu handhaben bzw. zu bearbeiten als in Wasser schwerlösliche oder nahezu wasserunlösliche Verbindungen. Insgesamt gesehen vereinfachen sich viele Probleme, die die Zubereitung und die Anwendung der Verbindungen I als Arzneistoff betreffen. Darüber hinaus ist ein wasserlöslicher Arzneistoff, wie die glatt in Wasser löslichen Salze der Verbindungen I, beispielsweise auch bezüglich der Dosierung leichter und sicherer anzuwenden.

In der nachstehenden Tabelle 1 sind zum Zweck des Vergleichs Löslichkeiten in Wasser von Salzen strukturell ähnlicher sekundärer Amide aufgeführt. Wie aus der Tab. 1 ersichtlich, handelt es sich bei den aufgeführten sekundären Amiden (Nr. 1-11) mit unterschiedlichen Substitutionsmustern im Phenylrest bis auf die wenig in Wasser lösliche Verbindung Nr. 6 durchweg um schwer und sehr schwer in Wasser lösliche Salze.

In der Tabelle 2 sind beispielhaft Löslichkeiten von in Wasser dagegen löslichen und leicht löslichen Salzen der erfindungsgemäßen Verbindungen I wiedergegeben.

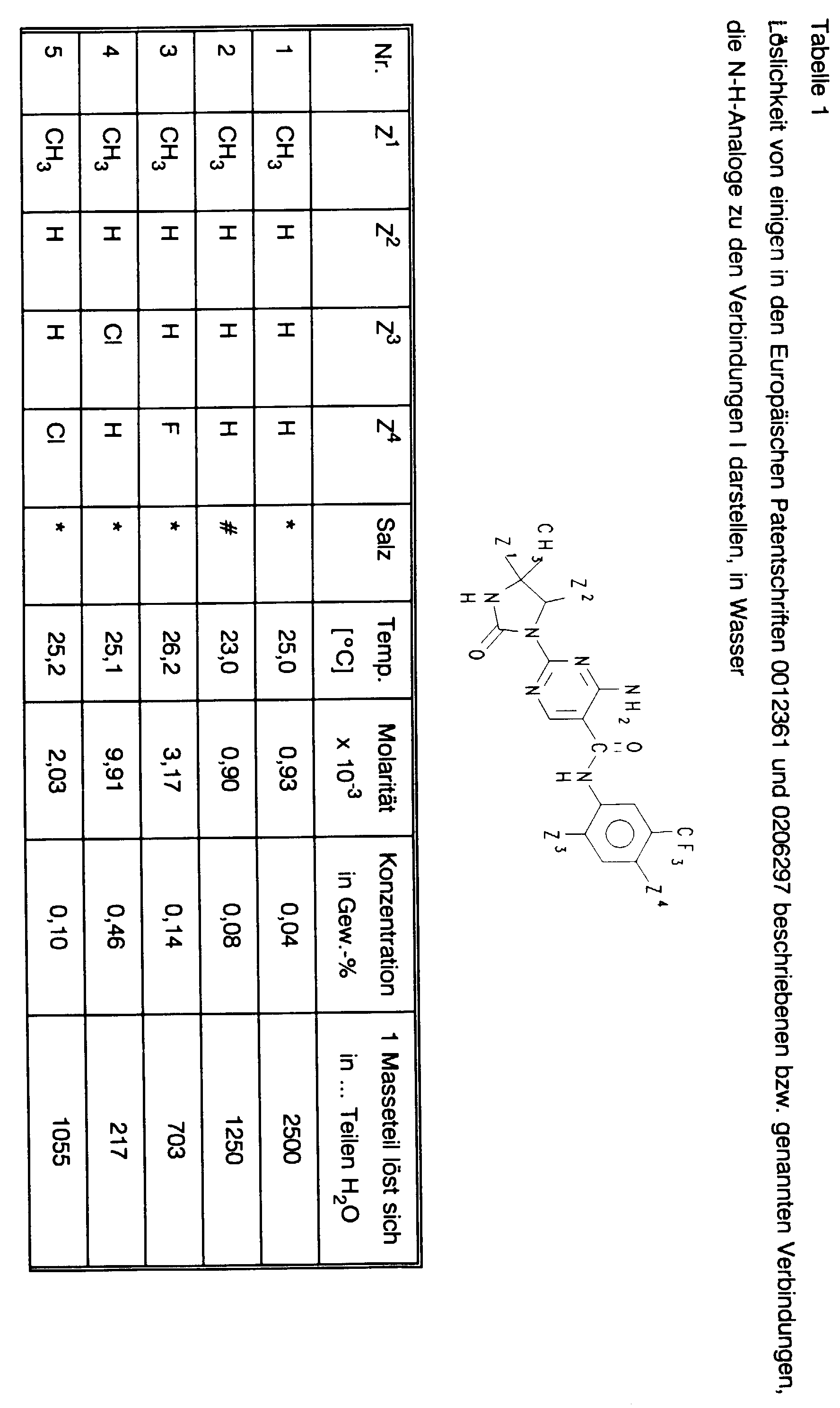

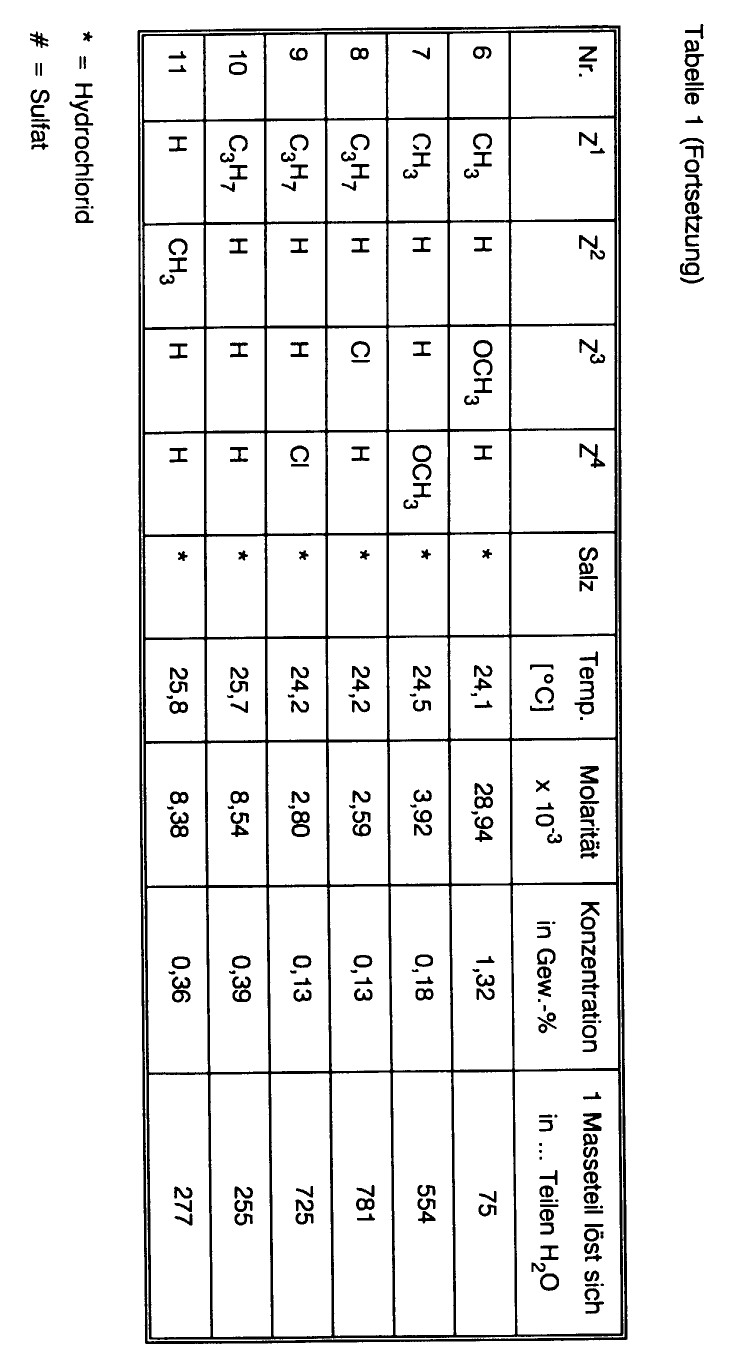

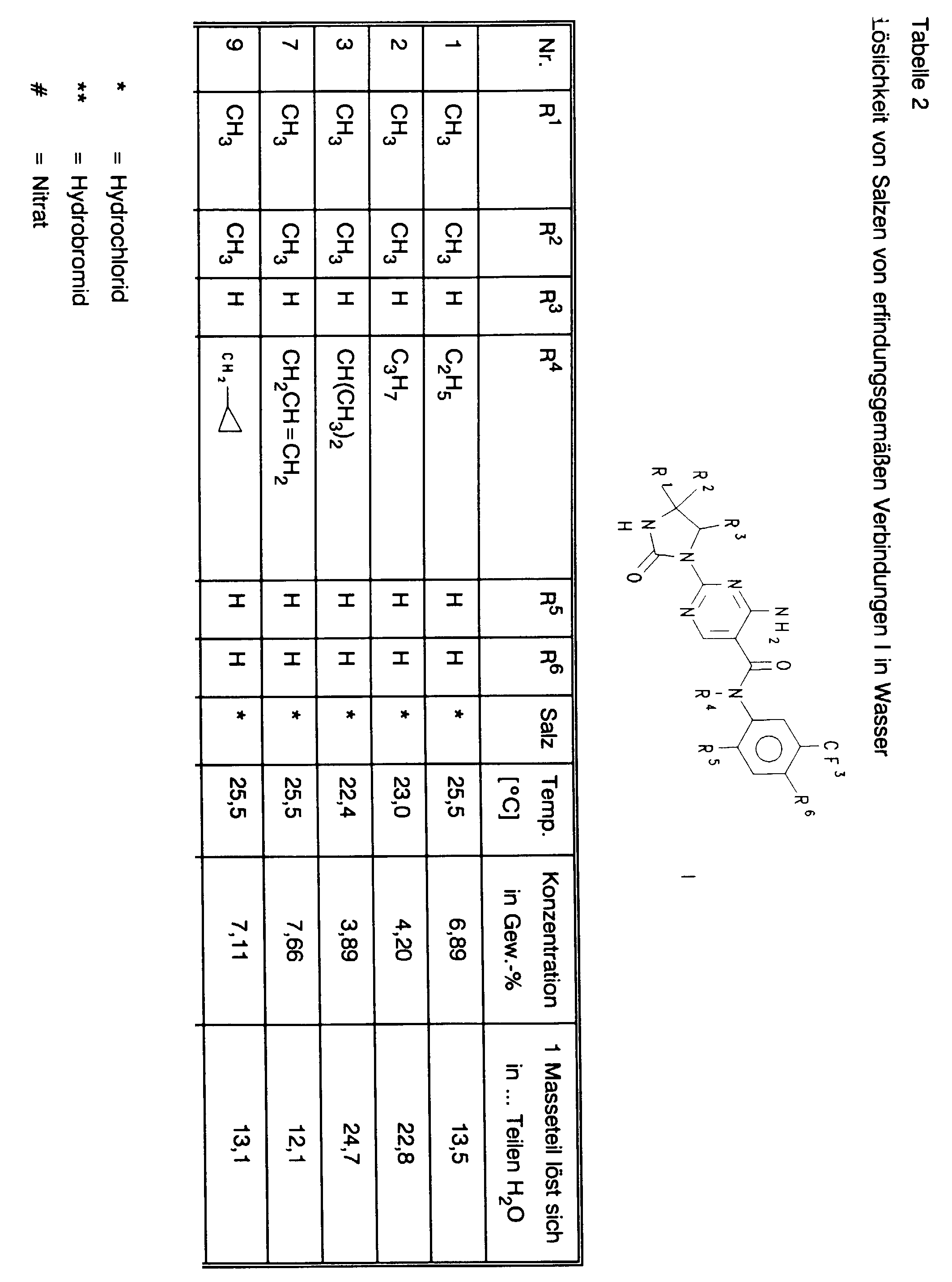

Die Erfindung betrifft weiterhin ein neues Verfahren zur Herstellung von 4-Amino-2-ureido-pyrimidin-5-carbonsäure-amiden der Formel I und von deren gut wasserlöslichen Salzen, dadurch gekennzeichnet, daß man eine Verbindung der Formel II, 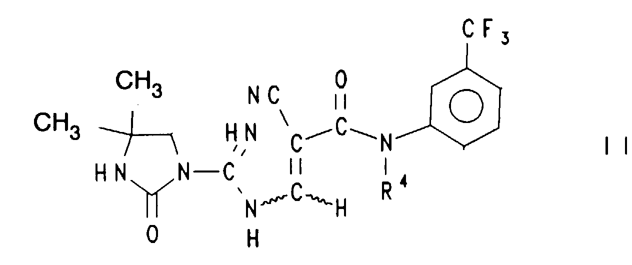 in der
R⁴, die zu Formel I angegebene Bedeutung hat, oder ein Salz oder ein Salzgemisch einer Verbindung II bei einer Temperatur von 0° bis 240°C mit oder ohne Lösemittelzusatz zu einer Verbindung der Formel I oder einem Salz oder einem Salzgemisch einer Verbindung I cyclisiert, eine erhaltene Verbindung der Formel I gegebenenfalls in ein in Wasser gut lösliches Salz überführt oder ein erhaltenes Salz oder Salzgemisch gegebenenfalls in ein in Wasser gut lösliches Salz überführt.

Die Guanidin-Derivate der Formel II sind neue Verbindungen. Die Erfindung betrifft daher auch die Verbindungen der Formel II in Form von beiden möglichen Stereoisomeren, der E- und der Z-Form, sowie ihre Säureadditionssalze.

Die Erfindung betrifft ferner ein Verfahren zur Herstellung der Verbindungen der Formel II oder von deren Salzen, das dadurch gekennzeichnet ist, daß man eine Verbindung der Formel III, 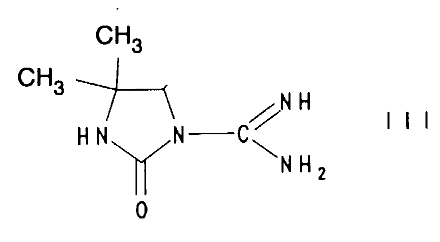 oder ein Salz dieser Verbindung III mit einer Verbindung der Formel IV, 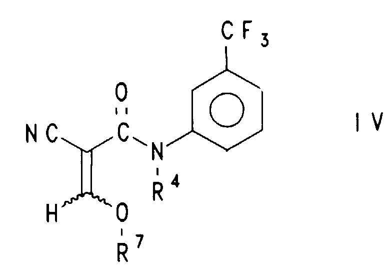 in der R⁴ die zu Formel I angegebene Bedeutung hat und R⁷ (C₁-C₄)-Alkyl bedeutet, in Gegenwart eines organischen Lose- oder Verdünnungsmittels unter Bildung einer Verbindung der Formel II oder einem Salz dieser Verbindung umsetzt, und die erhaltene Verbindung der Formel II gegebenenfalls in ein Salz überführt oder ein erhaltenes Salz gegebenenfalls in eine Verbindung der Formel II überführt.

Für den Fall, daß eine Verbindung III in Form eines Salzes angewandt wird, wird eine, vorteilhaft äquimolare, Menge einer basischen Verbindung bei der Umsetzung mit einer Verbindung der Formel III zusätzlich angewendet.

Die Verbindungen II fallen in der Regel als Stereoisomerenmischung (E und Z) an. Sie können aber auch als reine Stereoisomere (E- oder Z-Form) gebildet werden. Die Verbindungen II bzw. ihre Salze dienen als Ausgangsprodukte zur Herstellung der Verbindungen I bzw. deren gut wasserlöslichen Salzen. Die Verbindungen II bilden als einsäurige Basen Säureadditionssalze. Für deren Bildung sind im Prinzip alle Protonensäuren geeignet; vorteilhaft werden starke bis mittelstarke Säuren zur Salzbildung herangezogen. Als Beispiele seien folgende genannt: Chlor-, Brom-, Fluor- oder Jodwasserstoffsäure, Schwefel-, Phosphor-, Salpeter- oder Perchlorsäure, (C₁-C₄)- Alkan-phosphonsäuren, Sulfonsäuren wie Methan-, Benzol-, Toluol- oder Trifluormethan-sulfonsäure, Sulfaminsäure, Methylschwefelsäure, Essig-, Chloressig-, Dichloressig-, Trichloressig- oder Trifluoressigsäure, Ameisen-, Propion-, Oxal-, Malon-, Malein-, Bernstein-, Glutar-, Äpfel-, Wein-, Zitronen-, Fumar-, Milch-, Glykol- oder Brenztraubensäure, Benzoe- oder im Phenylrest substituierte Benzoesäuren wie Toluylsäure oder p-Nitro-benzoesäure oder Salicylsäure, Furancarbon- und/oder Mandelsäure.

Die als Ausgangsstoff zur Herstellung der Verbindung II erforderlichen Verbindungen III sind bekannt oder können nach beschriebenen Methoden hergestellt werden (DE-A-2853221).

Die Verbindungen der Formel IV können nach für diesen Enolether-Typ bekannten Methoden ausgehend von den entsprechenden N--Cyanacetyl-N-(cyclo)alkyl-N-(substituierten)phenyl-aminen hergestellt werden (vergl. z.B. Angew. Chemie Suppl. 1982, 1213 sowie Europäische Patentschrift 0012361). Die als Ausgangsstoffe zur Herstellung der Verbindungen IV erforderlichen N-Cyanacetyl-N-(cyclo)alkyl- bzw. --alkenyl-N-(substituierten)phenyl-amine werden nach bekannten Methoden (vgl. z.B. Britische Patentschrift No. 930808) hergestellt. Die zur Herstellung dieser Cyanacetylverbindungen notwendigen N-(Cyclo)alkyl-bzw. -alkenyl-aniline sind bekannt oder können nach bekannten Methoden, wie beispielsweise durch reduktive Alkylierung am Anilin-N oder durch Reduktion der entsprechenden Carbonamide mit LiAlH₄, gewonnen werden.

Unter einem Salz einer Verbindung III sind mit anorganischen sowie mit organischen Säuren zu erhaltende Säureadditionssalze zu verstehen. Bevorzugte Salze der Verbindungen III sind Hydrobromide, Hydrochloride oder Sulfate. Besonders geeignet für das erfindungsgemäße Verfahren sind Hydrobromide und/oder -chloride.

Die Umsetzung eines Amidino-imidazolidinons der Formel III mit einer Verbindung der Formel IV kann unter unterschiedlichsten Bedingungen erfolgen. Das betrifft sowohl die Reaktionstemperatur als auch gegebenenfalls mitzuverwendende Löse-bzw. Verdünnungsmittel. So kann die erfindungsgemäße Umsetzung der Verbindung III mit einer Verbindung IV bei einer Temperatur von -100°C bis +200°C mit oder ohne Zusatz eines Lose- bzw. Verdünnungsmittels erfolgen. Zweckmäßig wird diese Umsetzung von -30°C bis +100°C, vorzugsweise von +10°C bis +35°C, in Gegenwart von Lose- bzw. Verdünnungsmitteln durchgeführt.

Als Löse- bzw. Verdünnungsmittel sind im Prinzip alle gegenüber den Verbindungen III und IV (weitgehend) inerten Lösemittel, inklusive Wasser, brauchbar. Vorteilhaft werden C₁-C₅-Alkohole, Tetrahydrofuran, Dioxan, niedere aliphatische Ether, wie Diethyl-, Methyl-t.-butyl- sowie Diisopropylether, Acetonitril, N,N-Dimethylformamid, N,N-Dimethylacetamid, niedere Glykol- und Diglykol-di- und -monoether, wie 1,2-Dimethoxyethan, Methylglykol, Ethylglykol, Diglykoldimethylund -monomethylether, Ethylacetat, Methylacetat, Toluol, Pyridin, Aceton, Chloroform und/oder Dichlormethan als Löse- bzw. Verdünnungsmittel verwendet.

Bevorzugte Löse- bzw. Verdünnungsmittel sind C₂-C₄-Alkohole, Tetrahydrofuran, Dioxan, 1,2-Dimethoxyethan, Methyl-t.-butylether und/oder Acetonitril. Es können auch Mischungen der aufgeführten Lösungsmittel verwendet werden, sowie auch Mischungen der aufgeführten mit anderen Lösungsmitteln.

Bei der erfindungsgemäßen Umsetzung einer Verbindung III mit einer Verbindung IV können die (stöchiometrischen) Mengenverhältnisse, in denen diese Verbindungen angewandt werden, innerhalb eines breiten Bereichs schwanken. III und IV können in äquivalenten oder in nicht-äquivalenten Mengen miteinander umgesetzt werden. Letzteres bedeutet, daß eine der beiden Verbindungen in einem stöchiometrischen Überschuß angewendet werden kann. Bevorzugt wird die Verbindung III als Base mit Verbindung IV in äquivalenten Mengen oder in einem Verhältnis, wobei die Verbindung IV in einem stöchiometrischen Überschuß vorliegt, umgesetzt. Wird die Verbindung III als Säureadditionssalz angewandt, so kann auch sie gegenüber IV in einem stöchiometrischen Überschuß eingesetzt werden.

Die erforderlichen Reaktionszeiten sind von der Temperatur abhängig, bei der die Umsetzung durchgeführt wird. Sie können innerhalb eines breiten Bereichs schwanken. In der Regel betragen die Reaktionszeiten, wenn Temperaturen von +10°C bis +60°C angewandt werden, 0,3-10 Stunden, wobei die Reaktionszeiten - wie bekannt - im umgekehrten Verhältnis zur Temperatur stehen.

Wird die Verbindung III in Form eines Salzes angewandt, dann wird bei der erfindungsgemäßen Umsetzung - zweckmäßig in equimolarer Menge - eine basische Verbindung zugesetzt, bzw. die basische Verbindung III wird, bevor die Zugabe einer Verbindung IV erfolgt, mit einer basischen Verbindung aus ihrem Salz teilweise oder vollständig freigesetzt. Bevorzugt läßt man die zu diesem Zweck mit verwendete basische Verbindung vor Zugabe einer Verbindung der Formel IV auf das angewandte Salz des Amidino-imidazolidinons III einwirken. Die erfindungsgemäße Umsetzung kann somit auch in dieser Ausführungsform als "Eintopfreaktion" durchgeführt werden. Als basische Zusätze können anorganische und/oder organische basische Verbindungen verwendet werden. Als Beispiele seien genannt: Alkali- oder Erdalkalialkoholate von niederen Alkoholen, Alkali- oder Erdalkalihydroxide oder -hydride, als rein organische Basen tertiäre Amine, wie niedere Trialkylamine und/oder N,N-Dimethylanilin, Diazabicycloundecen (DBU) sowie analoge cyclische (trisubstituierte) Amidine, Tetraalkylammoniumhydroxide, 2-oder 4-Dialkylamino-pyridine und/oder Diazabicyclo-[2.2.2]-octan (Dabco).

Vorzugsweise werden Alkalialkoholate von (C₁-C₃)-Alkanolen, Natriumhydrid, DBU und DBU-analoge Amidine oder Tetramethyl-ammonium-hydroxid als basische Zusätze verwendet, wobei Natrium- oder Kaliummethylat und/oder -ethylat besonders geeignet sind.

Eine vorteilhafte Ausführungsform des erfindungsgemäßen Verfahrens besteht daher darin, daß eine Verbindung der Formel III in gelöster Form bei einer

Temperatur von 10°C bis 35°C mit einer Verbindung der Formel IV, in der R⁵, R⁶ und R⁷ die oben angegebene Bedeutung haben, wobei R⁷ bevorzugt Methyl oder Ethyl bedeutet, umgesetzt wird. Nach einer Reaktionszeit von 0,3-8 Stunden wird die gebildete, in der Regel als schwer lösliches Kristallisat abgeschiedene Verbindung der Formel II durch Abfiltrieren isoliert und bei einer Temperatur von 10°C bis 35°C - zweckmäßig unter Vakuum - getrocknet.

In Kenntnis dessen, was aus den eingangs zitierten europäischen Patentschriften bekannt ist, war es überraschend, daß bei der Umsetzung von einer Verbindung III mit einer Verbindung IV die jeweiligen Verbindungen II als kristalliner Feststoff in hohen Ausbeuten und hoher Reinheit isoliert werden können.

Bei der Herstellung der in der europäischen Patentschrift 0012361 beschriebenen Verbindungen sind Verbindungen vom Typ der Verbindung II nicht beobachtet worden. Insbesondere ist die Stabilität der Verbindungen II überraschend. Sie ermöglicht eine bequeme Isolierung dieser als Ausgangsstoff für das erfindungsgemäße Verfahren erforderlichen Verbindungen in guten Ausbeuten.

Die Isolierung der gebildeten Verbindungen der Formel II ist leicht möglich aufgrund der generellen Schwerlöslichkeit dieser Verbindungen, insbesondere in für das erfindungsgemäße Verfahren geeigneten, organischen Lösemitteln.

Da die Verbindungen II im Rahmen der vorliegenden Erfindung zu einem Endprodukt der Formel I bzw. zu gut löslichen Salzen dieser Verbindungen I umgesetzt werden, ist es nicht zwingend erforderlich, die Verbindungen II bzw. deren Salze in reiner Form zu isolieren. Vielmehr kann die Cyclisierung unter Änderung der Reaktionsbedingungen im Sinne einer Eintopfreaktion durchgeführt werden.

Die Cyclisierung der Verbindungen II als Base kann mit oder ohne Zusatz eines Löse- oder Verdünnungsmittels erfolgen. Die Cyclisierung in Gegenwart von Löse-bzw. Verdünnungsmitteln wird bei einer Temperatur von 0°C bis 130°C, vorzugsweise von 40°C bis 95°C, ausgeführt. Die Cyclisierung ohne Löse- bzw. Verdünnungsmittelzusatz wird bei einer Temperatur von 60°C bis 240°C, vorzugsweise von 160°C bis 200°C, durchgeführt.

Wird eine Verbindung II als Base unter Bildung einer Verbindung I cyclisiert, dann besteht eine Ausführungsform darin, daß man die zweckmäßigerweise trockene Verbindung der Formel II auf eine Temperatur von 160°C bis 200°C 3-27 Minuten - vorzugsweise 10-20 Minuten - lang erhitzt und danach sofort auf Raumtemperatur abkühlt. Anschließend wird die bei der thermischen Cyclisierung aus II gebildete Verbindung I isoliert. Das beinhaltet in der Regel die Abtrennung von zwei in spürbaren Mengen mitentstandenen Nebenprodukten, dem 3-(Cyclo)alkyl- bzw. Alkenyl-aminobenzotrifluorid (Formel V im Reaktionsschema I) und der Verbindung der Formel VI (vgl. Schema I). Die Verbindung VI kann leicht aufgrund ihrer Löslichkeit in verdünnten wäßrigen Alkalien und die Verbindung der Formel V größtenteils aufgrund ihrer größeren Flüchtigkeit destillativ von der Verbindung I abgetrennt werden. Die vollständige Abtrennung der Verbindung V von der Verbindung I ist einfach möglich durch Umkristallisieren und/oder durch Auswaschen der kristallinen Verbindung I mit solchen Lösemitteln, die die Verbindung I schwer und die Verbindung V leicht lösen.

Die Cyclisierung einer Verbindung II in Form eines Säureadditionssalzes wird vorzugsweise unter Mitverwendung von Löse- bzw. Verdünnungsmitteln bei einer Temperatur von 0°C bis 150°C, vorzugsweise von 45°C bis 95°C, ausgeführt. Diese Cyclisierung eines Salzes der Verbindung II kann aber auch ohne Zusatz eines Löse- oder Verdünnungsmittels bei einer Temperatur von 60°C, vorzugsweise 160°C, bis 240°C, vorzugsweise 200°C, ausgeführt werden.

Für diese Art der Cyclisierung der Verbindungen II, mit oder ohne Lösemittelzusatz, können definierte, gesondert aus einer Verbindung II hergestellte Säureadditionssalze sowie auch Mischungen, bestehend aus einer Verbindung II und einer oder mehrerer Säuren verwendet werden. Für solche Mischungen sind alle Zusammensetzungen, beginnend bei minimalsten Mengen an Säureäquivalenten, z.B. kleiner als 10⁻⁴ Äquivalenten, bis zu 2 Säureäquivalenten oder mehr verwendbar; vorzugsweise werden 0,8-1,6 Säureäquivalente angewandt. Im Extremfall kann die Cyclisierung einer Verbindung II in einer Säure, die gleichzeitig als Löse- bzw. Verdünnungsmittel dient, durchgeführt werden. Diese Ausführungsform ist bevorzugt, falls Ameisen-, Essig- und/oder Propionsäure als Säuren und als Verdünnungsmittel verwendet werden. Bei der Cyclisierung von Säureadditionssalzen der Verbindungen II, unter Bildung einer Verbindung I, ist also nicht zwingend der Einsatz von definierten, separat hergestellten Salzen erforderlich.

Die Cyclisierung einer Verbindung II unter Zusatz einer oder mehrerer Säuren in Gegenwart von Löse- bzw. Verdünnungsmitteln ist eine gut geeignete Ausführungsform des erfindungsgemäßen Verfahrens.

Eine vorteilhafte Ausführungsform der Cyclisierung von Salzen einer Verbindung II bzw. der Cyclisierung einer Verbindung II unter Zusatz einer oder mehrerer Säuren besteht darin, daß man eine Verbindung II unter Zusatz von 0,2 bis 2,5 oder mehr - vorzugsweise 1 bis 1,5 - Säureäquivalenten in Gegenwart eines Lose- bzw. Verdünnungsmittels auf eine Temperatur von 25°C bis 120°C, vorzugsweise von 45°C bis 95°C, erwärmt. Die Reaktionszeiten betragen dabei, in Abhängigkeit von der Temperatur, 0,5 - 9 Stunden. Anschließend kann durch Zusatz von geeigneten Lösemitteln, worin das entsprechende Salz der gebildeten Verbindung I schwer löslich ist, dieses kristallin abgeschieden werden. Auf diese Weise gelingt eine einfache und effektive Abtrennung einer gebildeten Verbindung I von mitentstandenen Nebenprodukten (vgl. dazu das Reaktionsschema I).

Für die vorstehend beschriebene Ausführung der Cyclisierung von Verbindungen II sind im Prinzip alle Protonensäuren brauchbar. Als Beispiele seien folgende genannt: Chlor-, Brom-, Fluor- oder Jodwasserstoffsäure, Schwefel-, Phosphor-, Salpeter- oder Perchlorsäure, (C₁-C₄)-Alkan-phosphonsäuren, Sulfonsäuren wie Methan-, Benzol-, Toluol- oder Trifluormethan-sulfonsäure, Sulfaminsäure, Methylschwefelsäure, Essig-, Cyanessig-, Chloressig-, Dichloressig-, Trichloressig-oder Trifluoressigsäure, Ameisen-, Propion-, Oxal-, Malon-, Malein-, Bernstein-, Glutar-, Äpfel-, Wein-, Zitronen-, Fumar-, Milch-, Glykol- oder Brenztraubensäure, Benzoe- oder im Phenylrest substituierte Benzoesäuren wie Toluylsäure oder p-Nitro-benzoesäure oder Salicylsäure, Furancarbon- und/oder Mandelsäure.

Bevorzugt werden Essig-, Oxal-, Wein-, Ameisen-, Zitronen-, Äpfel-, Fumar-, Dichloressig-, Trichloressig- und/oder Propionsäure angewandt.

Besonders geeignet sind Essig-, Oxal-, Wein-, Ameisen-, und/oder Zitronensäure.

Als Löse- bzw. Verdünnungsmittel für diese Ausführung der Cyclisierung einer Verbindung II in Form von Salzen oder Salzgemischen sind im Prinzip alle gegenüber den Verbindungen II inerten organischen Lösemittel und/oder Wasser geeignet. Vorteilhaft werden C₁-C₄-Alkohole, Tetrahydrofuran, Dioxan, Methyl-t.-butylether, Acetonitril, N,N-Dimethylform- und/oder -acetamid, niedere Glykol-di- und -monoether, wie 1,2-Dimethoxyethan, Methyl- und/oder Ethylglykol, und/oder Methylacetat, Ethylacetat, Aceton, Chloroform, Dichlormethan, Ameisensäure und/oder Essigsäure verwendet.

Eine bevorzugte Ausführungsform des erfindungsgemäßen Verfahrens besteht darin, daß eine Verbindung II in Gegenwart von Eisessig und/oder Ameisensäure, mit oder ohne Zusatz einer oder mehrerer mittelstarken bis starken Säure(n) 0,3 bis 5 Stdn. auf eine Temperatur von 25°C bis 120°C, vorzugsweise von 45°C bis 95°C, erwärmt wird.

Eisessig und/oder Ameisensäure dienen dabei zugleich als Lösemittel und werden, bezogen auf die jeweilige Verbindung II, in einem stöchiometrischen Überschuß angewandt. Von den ggfs. mitzuverwendenden mittelstarken bis starken Säuren seien Oxal-, Citracon-, Wein-, Zitronen-, Trifluoressig-, Cyanessig-, Chloressig-, Dichloressig- und/oder Trichloressigsäure und/oder Polyphosphorsäure beispielhaft genannt.

Im Bedarfsfall wird eine Verbindung I aus dem isolierten Salz in für solche Basenbildungen allgemein bekannter Weise freigesetzt und ggfs. durch Zusatz einer entsprechenden Säure in ein (anderes) in Wasser gut lösliches Salz übergeführt.

Die im Zusammenhang mit der Salzbildung der Verbindungen I genannten "Säureäquivalente" beziehen sich auf die Verbindungen I als einsäurige Basen.

Das erfindungsgemäße Verfahren zur Herstellung der Verbindungen I wird durch nachstehendes Formelschema erläutert.

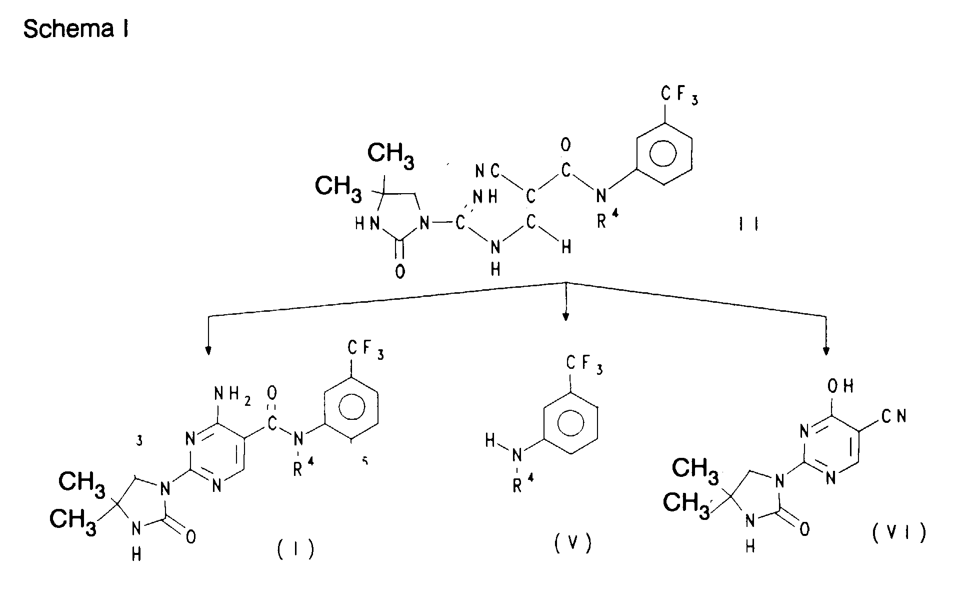

Ein bei der Cyclisierung einer Verbindung II als Nebenprodukt gebildetes 3-(Cyclo)alkyl- bzw. -alkenyl-amino-benzotrifluorid V kann isoliert und in den Herstellungsprozeß der entsprechenden Verbindung IV zurückgeführt werden.

Die Verbindungen der Formel I und die in Wasser gut löslichen Salze dieser Verbindungen besitzen wertvolle pharmakologische Eigenschaften und können daher als Arzneimittel verwendet werden. Sie zeichnen sich beispielsweise durch hypolipidämische Eigenschaften aus.

Es wurde überraschenderweise gefunden, daß die Verbindungen der Formel I und die in Wasser gut löslichen Salze dieser Verbindungen LDL-Rezeptor-Level erhöhen. Die Verbindungen I und ihre in Wassr gut löslichen Salze sind daher zur Behandlung von Lipidstoffwechselstörungen, die durch eine Stimulierung des hepatischen LDL-Rezeptors günstig beeinflußt werden können, geeignet. Die Erfindung betrifft daher auch die Verwendung von Verbindungen I und von deren in Wasser gut löslichen Salzen zur Behandlung der genannten Lipidstoffwechselstörungen.

Die Wirkung beruht auf einer raschen direkten Stimulation des LDL-Rezeptors (apoB/E Rezeptor) in der Leber. Über die Stimulation des natürlichen Cholesterin-Katabolismuswegs senken die Verbindungen I und ihre in Wasser gut löslichen Salze das atherogene LDL und VLDL. Die HMG-CoA Reduktase Inhibitoren oder lonenaustauscherharze hingegen stimulieren durch Absenkung der intrazellulären Cholesterinspiegel bzw. durch Erhöhung des Cholesterinbedarfs für die Biosynthese von Gallensäuren indirekt die Expression der LDL-Rezeptoren auf der Leberzelle. Somit sind Nebenwirkungen, wie sie bei den Statinen, bedingt durch ihre drastische Inhibition der HMG-CoA Reduktase auch in anderen Organen als der Leber zu beobachten sind (z.B. Beeinflussung der Ubichinonbildung), nicht zu erwarten. Solche Nebenwirkungen wurden bislang auch nicht beobachtet. Compliance-Probleme wie bei den Ionenaustauscherharzen, die durch die erforderlichen großen täglichen Dosen von 15 bis 30 g hervorgerufen werden, sind nicht zu befürchten.

Besonders gefährlich bei der Atherogenese sind bestimmte cholesterintransportierende Lipoproteinfraktionen wie die kleinpartikulären LDL-Fraktionen. Der Katabolismus des überwiegenden Teils des LDL (75 %) erfolgt durch den LDL-Rezeptor in der Leber. In den Leberzellen wird das Cholesterin dann teilweise zu Gallensäuren metabolisiert und in Form von Galle exkretiert, teilweise erfolgt auch eine direkte Ausscheidung von Cholesterin über die Galle. Es ist somit im Prinzip bereits anerkannt und bereits therapeutisch durch die indirekt wirksamen Substanzen (wie Statine) belegt, daß der LDL-Rezeptor ein idealer Angriffspunkt für ein hypolipidämisch wirksames Antiatherosklerotikum beim Menschen ist.

Die Verbindungen I und ihre in Wasser gut löslichen Salze erhöhen den LDL-Rezeptor-Level. Die erhöhte LDL- (IDL-, Chylomikronen Remnant-) Aufnahme in die Leberzellen, bedingt durch die so erhöhten LDL-Rezeptor-Level auf der Zelloberfläche, führt dann zu einem verstärkten hepatischen Abbau des LDL-Cholesterins. Die HDL-Spiegel werden nicht beeinflußt.

Die Verbindungen der Formel I und die in Wasser gut löslichen Salze stellen somit ein ideales antiatherosklerotisch wirksames Prinzip dar. Folgende Befunde belegen den aufgefundenen Wirkmechanismus, der die beschriebenen Verbindungen als besonders geeignet für die Behandlung solcher Hyperlipidämien, die auf einer verminderten LDL-Rezeptor-Aktivität beruhen, erscheinen läßt:
1a. In der allerseits als Modell anerkannten humanen Hepatozytom-Zellinie HepG2 werden die LDL-Rezeptor mRNA Spiegel durch die Verbindungen der Formel I erhöht (Tabelle I).
1b. Auch in Rattenlebern werden innerhalb weniger Stunden die LDL-Rezeptor mRNA Level durch Verbindungen der Formel I gesteigert (Tabelle II).
Die Stimulierung liegt im Bereich von 170 bis 350 % der Kontrollen (Kontrolle = 100 %).
Die Präparation der mRNA wurde nach der Methode von Chomczynski, P. und Sacchi, N., Anal. Biochem. 162, 156 - 159 (1987) durchgeführt. Bei Organen (wie z.B. Leber) wurde das tiefgefrorene Gewebe auf Trockeneis zuvor im Mörser homogenisiert und die mRNA mittels Oligo dT nach Standardmethoden weiter angereichert (vgl. Sambrook, J., Fritsch, E. F. und Maniatis, T., Molecular Cloning, zweite Auflage, Cold Spring Harbor (1989); in dieser Methodensammlung finden sich auch Beschreibungen aller weiteren hier verwendeten relevanten molekularbiologischen Standardverfahren). 5 bis 20 µm der so gewonnenen gelösten mRNA wurden nach Standardverfahren denaturiert und auf 1 %igen horizontalen Agarosegelen aufgetrennt. Die mRNA wurde mittels Kapillarblot auf Hybond N Membranen (Amersham) transferiert. Als spezifische Hybridisierungsprobe diente ein partieller LDL-Rezeptor cDNA Klon und als interner Standard ein Plasmid, das ein β-Aktin Gen enthielt. Beide Plasmide wurden mittels eines random Primer Kit von Amersham bis zu einer spezifischen Aktivtät von 5 x 10⁹ cpm/µg markiert. Vorhybridisierung, Hybridisierung und Waschen der Filter erfolgten nach Standardverfahren. Die Filter wurden anschließend auf Cronex 4 Filmen (Dupont) über Nacht bis zu 14 Tagen in Gegenwart einer Verstärkerfolie bei -70°C exponiert und die Hybridisierungssignale über die Filmschwärzungsintensität mit einem handelsüblichen Laserdensiometer quantifiziert. Anschließend wurde der Quotient aus der Intensität der LDL-Rezeptorbande und der Aktinbande als internem Standard zur Korrektur von Ausbeutevariationen bestimmt.
Tabelle I gibt die Stimulierung der LDL-Rezeptor-mRNA-Expression auf HepG2 Zellen durch ausgewählte Verbindungen der Formel I im Vollserum (Endkonzentration der Verbindungen 10⁻⁶ M) nach einer 16 h Inkubation wieder. Die HepG2 Zellen wurden in RPMI 1640 Standardmedium mit fötalem Kälberserum (Endkonzentration 10 %) inkubiert. Als Induktionskontrolle diente serumfreies RPMI Medium. Anschließend wurde die gesamt mRNA präpariert und mittels der Northern-Blot-Technik die relevanten LDL-Rezeptor-mRNA und β-Aktin-mRNA Level bestimmt. Der Quotient aus dem LDL-Rezeptor-mRNA Signal und dem β-Aktin-mRNA Signal der Kontrolle (ohne Substanzzugabe) wurde als 100 % gesetzt und die darüber unter dem Einfluß der Verbindungen erreichte Stimulierung der LDL-Rezeptor-mRNA Level in Prozent der Kontrolle ausgedrückt.

**Tabelle I**

| Verbindungen gemäß Beispiel | Konzentr. | LDL-Rezeptor-mRNA |
|---|---|---|
| 2 | 2x10⁻⁶ M | 175 % |
| 4 | 2x10⁻⁶ M | 205 % |
| 12 | 2x10⁻⁶ M | 170 % |
| 33 | 2x10⁻⁶ M | 205 % |
| 40 | 2x10⁻⁶ M | 180 % |

Tabelle II zeigt die Stimulierung der LDL-Rezeptor-mRNA-Expression in Rattenlebern 6 Stunden nach einer Applikation ausgewählter Verbindungen der Formel I (Dosis von 30 mg/kg). Lebergewebe wurde entnommen und in flüssigem Stickstoff schockgefroren.
Anschließend wurde die mRNA wie beschrieben isoliert und mittels der Northern-Blot-Technik die relativen LDL-Rezeptor-mRNA-Level bestimmt. mRNA Level von unbehandelten Kontrolltieren wurden als 100 % gesetzt und die Stimulierung der LDL-Rezeptor-mRNA in Prozent der Kontrolle errechnet.

**Tabelle II**

| Verbindungen gemäß Beispiel | Konzentr. | LDL-Rezeptor-mRNA |
|---|---|---|
| 2 | 30 mg/kg | 300 % |
| 4 | 30 mg/kg | 221 % |
| 33 | 30 mg/kg | 350 % |
| 40 | 30 mg/kg | 340 % |

2. Bei der HMG-CoA-Reduktase-Bestimmung in vitro an teilgereinigter HMG-CoA-Reduktase aus Rattenleber (vgl. Avigan J., Bathena, S.J., und Schreiner, M.E., J. Lipid Res. 16, 151 (1975) und Philippi, B.W., und Shapiro, D.J., J. Lipid Res. 20, 588 (1979)) beeinflussen die Hydrochloride von Verbindungen I bei 10⁻⁵ M Zusatz (Endkonzentration) gegenüber Kontrollinkubationen die Enzymaktivität nicht (Streuung 10 %), Lovastatin-Na (mit geöffnetem Lactonring) hat in diesem Versuch eine IC₅₀ bei 3 x 10⁻⁹ M.
3. In vivo spiegelt sich die erhöhte LDL-Rezeptoraktivität bei Versuchstieren in einer Absenkung der durch den LDL-Rezeptor (apoB/E Rezeptor) metabolisierbaren Lipoproteinfraktionen wieder, d.h. es wird eine Senkung der LDL- und VLDL-Spiegel beobachtet. Hierzu wurde der folgende Test durchgeführt:
Wirkung auf Serumlipoproteine normolipämischer männlicher Ratten im subchronischen Versuch
Gruppen von jeweils 10 männlichen Ratten des Stammes HOE:WISKf (SPF 71) mit einem Ausgangsgewicht über 180 g, erhielten täglich einmal (morgens) das Prüfpräparat bzw. das Vergleichspräparat in 1 % wäßriger ®Tylose MH 300-Lösung per Magensonde (0,5 ml/100 g Körpergewicht); die jeweilige Kontrollgruppe erhielt nur das Vehikel. Die letzte (7.) Applikation erfolgte 24 Stunden vor Blutentnahme und Tötung. Zu Futter und Wasser bestand freier Zugang während des Versuches. 24 Stunden vor der Blutentnahme wurde das Futter entzogen.
Zur Analyse der Serum-Lipoproteine wurde das Serum aller 10 Ratten einer Gruppe gepoolt. Die Serum-Lipoproteine wurden mit der präparativen Ultrazentrifuge (KONTRON TGA 65, Rotor BECKMAN 50.4 Ti) getrennt.
Folgende Bedingungen gemäß Koga, S., Horwitz, D.L., und Scanu, A.M., Journal of Lipid Research 10, 577 (1969) und Havel, R.J., Eder, H.A. und Bragdon, H.H., J. Clin. Invest. 34, 1345 (1955) wurden für die Trennung von VLDL und LDL verwendet:
1. VLDL: Dichte < 1,006 16 Stunden bei 40 000 Upm
2. LDL: Dichte 1,006 - 1,04 16 Stunden bei 40 000 Upm

Zur enzymatischen Bestimmung des Cholesterins und der Triglyceride in den getrennten Lipoproteinfraktionen wurden Testkombinationen von BOEHRINGER/Mannheim (vgl. Siedel, J., Schlumberger, H., Klose, S., Ziegenhorn, J. und Wahlefeld, A.W., J. Clin. Chem. Clin. Biochem. 19, 838 (1981) und Wahlefeld, A.W., in: H.O. Bergmeier: Methoden der enzymatischen Analyse, 2. Auflage, Band II, Verlag Chemie 1974, S. 1878) verwendet. Die Proteine wurden nach Lowry (Lowry, O. H., Roseborough, N. H., Farr, A.L. und Randell, R. J., J. Biol. Chem. 193, 265 (1951)) bestimmt.
Die Ergebnisse sind in Tabelle III zusammengefaßt. Als Vergleichspräparat diente Clofibrat.

**Tabelle III**

| | | Serum % Veränderung gegen Kontrolle am Behandlungsende | | | | |
|---|---|---|---|---|---|---|
| Verb. gemäß Beisp. | Dosis mg/kg/Tag | Cholesterin | | Protein | | Triglyceride |
| | | VLDL | LDL | VLDL | LDL | VLDL |
| 2 | 30 | -96 | -75 | -40 | -73 | -81 |
| | 10 | -41 | -32 | -28 | -28 | -26 |
| | 3 | -30 | -40 | -7 | -28 | -40 |
| | 1 | -22 | -28 | | -44 | |
| 4 | 30 | -70 | -68 | -13 | -53 | -29 |
| | 10 | +2 | -39 | -18 | -33 | |
| 12 | 30 | -80 | -66 | -51 | -30 | -29 |
| | 10 | -63 | -46 | -34 | -18 | -51 |
| | 3 | -6 | -26 | -8 | -5 | -4 |
| 32 | 30 | -100 | -76 | -39 | -49 | -80 |
| | 10 | -85 | -54 | -73 | -36 | -68 |
| 33 | 30 | -95 | -67 | -33 | -44 | -100 |
| | 10 | -48 | -47 | -29 | -33 | -73 |
| 35 | 30 | -79 | -46 | -69 | -35 | -55 |
| 40 | 30 | -57 | -58 | -52 | -52 | -41 |
| | 10 | -40 | -53 | -41 | -38 | -2 |
| | 3 | -19 | -22 | -11 | -18 | |
| 41 | 30 | -100 | -66 | -45 | -36 | -80 |
| | 10 | -100 | -73 | -52 | -48 | -68 |
| 58 | 30 | -59 | -46 | -22 | -25 | -40 |
| Clofibrat | 100 | -51 | -31 | -30 | -32 | -34 |

4. Vergleichende antiproliferative Untersuchungen:
Exponentiell wachsende Tumorzellen (Mäuseleukämie-Zellen, L1210; Bronchialkarzinom-Zellen, A549; Kolonkarzinom-Zellen, HT29) werden in einer Konzentration von 5 x 10³ Zellen pro ml in RPMI Standard Medium in 96 Loch Mikrotiterplatten ausgesäht. Die Inkubation mit Testsubstanz-Konzentrationsreihen erfolgt 72 Stunden bei 37°C, 5 % CO₂, 95 % rel. Luftfeuchtigkeit. Jede Verbindungskonzentration bzw. Kontrolle wird dabei in vier Parallelinkubationen getestet. Nach 65 Stunden werden 50 µl MTT [3-(4,5-Dimethyl-2-thiazolyl)-2,5-diphenyl-2H-tetrazolium-bromid] in 2,5 mg/ml PBS zugesetzt. In intakten Zellen wird MTT reduziert zu einem roten unlöslichen Farbstoff. In Abhängigkeit von der verwendeten Zellinie wird nach weiteren 7 bis 24 Stunden Inkubation der Überstand entfernt. Der entstandene unlösliche Farbstoff wird in 100 µl DMSO unter vorsichtigem Schütteln gelöst und die Extinktion in einem Multiscan Photometer 340 CC der Fa. Flow bei 492 nm gemessen.
Die Ergebnisse werden als Quotienten aus den gemittelten Extinktionswerten der Testsubstanzen und der Kontrollwerte berechnet. Die Schwankungen der Einzelbestimmungswerte innerhalb der Parallelwerte sind kleiner als 15 %. Aus Dosis-Wirkungsdiagrammen wird für die gegebenen Verbindungen der IC₅₀ Wert abgelesen.

Die Verbindungen der Formel I können in Form ihrer pharmazeutisch annehmbaren Säureadditionssalze - diese Verwendungsform wird bevorzugt - oder als freie Base aufgrund ihrer pharmakologischen Eigenschaften als Arzneimittel Verwendung finden, wobei man sie entweder allein oder vermischt mit geeigneten Trägerstoffen und/oder verträglichen Verdünnungsmitteln und ggfs. auch noch mit anderen Zusätzen verabreicht.

Die vorliegende Erfindung betrifft auch pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen inerten pharmazeutischen geeigneten Trägerstoffen sind pharmazeutisch unbedenkliche feste, halbfeste oder flüssige Verdünnungsmittel, Füllstoffe und Formulierungshilfsmittel jeder Art zu verstehen, die nach dem Vermischen mit dem Wirkstoff diesen in eine für die Verabreichung geeignete Form bringen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, ggfs. sterile injizierbare Lösungen, nichtwäßrige Emulsionen, Suspensionen und Lösungen, Sprays sowie Zubereitungsformen mit protrahierter Wirkstoff-Freigabe in Betracht.

Die therapeutisch wirksamen Verbindungen sollen in den oben aufgeführten pharmazeutischen Zubereitungen zweckmäßig in einer Konzentration von etwa 0,1, vorzugsweise von 0,5, bis 99,0, vorzugsweise bis 70,0, Gewichtsprozenten der Gesamtmischung vorhanden sein.

Die Anwendungskonzentrationen für Lösungen sowie Aerosole in Form von Spray betragen im allgemeinen 0,1 bis 20, vorzugsweise 0,5 - 5, Gewichtsprozent.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen. In den Beispielen (118 und 119) sind geeignete Tablettenzusammensetzungen beschrieben.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Humanmedizin zur Verhütung, Besserung/und oder Heilung der oben angeführten Erkrankungen.

Die Wirkstoffe oder die pharmazeutischen Zubereitungen können oral, parenteral, intraperitoneal und/oder rectal appliziert werden.

Die z.B. als Hypolipidämika verwendbaren Verbindungen der vorliegenden Erfindung, vorzugsweise ihre Salze, können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Kapseln (Gelatinekapseln), welche den Wirkstoff zusammen mit Verdünnungsmitteln bzw. Trägerstoffen, z.B. Lactose, Dextrose, Rohrzucker, Mannitol, Sorbitol, Cellulose, verschiedenen Stärkearten und/oder Glycin, und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumcarbonat, Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wässrige Lösungen oder Suspensionen, die sterilisiert sein können und Hilfsstoffe, wie Konservier-, Stabilisierungs-, Netzund/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die wenn erwünscht weitere pharmakologisch wirksame Stoffe enthalten können, werden z.B. mittels konventioneller Misch-Granulier- und Dragierverfahren, hergestellt und enthalten 0,1 % bis vorzugsweise 80 %, bevorzugt etwa 5 % bis etwa 65 %, des Wirkstoffs.

Die orale Anwendung erfolgt in pharmazeutisch üblichen Zubereitungen, beispielsweise in Form von Tabletten, Dragees oder Kapseln, die z.B. pro Tagesdosis 5 bis 1000 mg, vorzugsweise 20 bis 200 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die nachfolgenden Beispiele dienen zur näheren Erläuterung der Erfindung, ohne dieselbe auf in den Beispielen beschriebenen Produkte und Ausführungsformen einzuschränken.

Die Temperaturen sind in °C angegeben.

Die in den nachfolgenden Beispielen angegebenen Schmelz- bzw. Zersetzungspunkte sind unkorrigiert.

Dünnschichtchromatographien wurden auf DC-Fertigplatten, Kieselgel 60, F-254 der Firma Riedel-de Haen AG, und, sofern in den Beispielen kein anderes Fließmittel angegeben ist, mit dem Fließmittel CH₂Cl₂/C₂H₅OH 10:1 (v/v) ausgeführt.

### Referenzbeispiele

### Referenzbeispiel 5

Eine Suspension von 16,70 g (37 mMol) einer Verbindung II, in der R¹ und R² CH₃, R³, R⁵ und R⁶ Wasserstoff und R⁴ n-Butyl bedeuten, in 41 ml Eisessig wurde 4 Stunden bei 60° gerührt, wobei eine Lösung entstand. Diese dampfte man im Vakuum ein. Den Rückstand nahm man in 300 ml CH₂Cl₂ auf und schüttelte diese Lösung dreimal mit je 40 ml 2 N NaOH und zweimal mit je 50 ml Wasser aus. Nach Trocknen und Filtrieren wurde die CH₂Cl₂-Lösung im Vakuum eingedampft. Der verbleibende ölige Rückstand (16,5 g) wurde in 15 ml Ethylacetat gelöst und mit Ether bis zur beginnenden Trübung versetzt, wonach Kristallisat ausfiel. Nach 2 Stunden Rühren im Eisbad wurde der Feststoff abgesaugt, mit Ether ausgewaschen und 20 Stunden bei 100° getrocknet. Man erhielt 9,48 g (56,8 % Ausbeute) reines 4-Amino-2-(4,4-dimethyl-2-oxo-imidazolidin-1-yl)-pyrimidin-5-carbonsäure-(N-butyl-N-(3-trifluormethyl-phenyl)-amid), Fp. 154-155°.

| C₂₁H₂₅F₃N₆O₂ (450,48) | | | | |
|---|---|---|---|---|
| berechnet: | C 55,99 | H 5,59 | F 12,65 | N 18,66 % |
| gefunden: | C 55,3 | H 5,6 | F 12,5 | N 18,4 % |

### Referenzbeispiel 6

Eine Lösung von 4,96 g (11 mMol) der gemäß voranstehendem Beispiel hergestellten Verbindung I (R¹, R² = CH₃; R³, R⁵, R⁶ = H; R⁴ = n-C₄H₉) in 25 ml Aceton wurde bei Raumtemperatur mit 1,9 ml einer 6,17 molaren Lösung von HCI in Ether versetzt. Die entstehende Suspension rührte man 2 Stunden unter Eiskühlung. Danach wurde das Kristallisat abgesaugt, mit Aceton und Ether gewaschen und 15 Stunden 100° im Vakuum (6 mbar) getrocknet. Man erhielt 5,44 g (∼ 100 %) reines 4-Amino-2-(4,4-dimethyl-2-oxo-imidazolidin-1-yl)-pyrimidin-5-carbonsäure-(N-butyl-N-(3-trifluormethyl-phenyl)-amid)-hydrochlorid, Fp. 266-267° (Zers.).

| C₂₁H₂₆CIF₃N₆O₂ (486,94) | |
|---|---|
| berechnet: | Cl^{⊖} 7,28 % |
| gefunden: | Cl^{⊖} 7,3 % |

### Referenzbeispiel 43

Eine Mischung aus 2,86 g (7 mMol) einer Verbindung II (R¹ = CH₃; R², R³, R⁵, R⁶ = H; R⁴ = C₂H₅), 5 ml Eisessig und 0,70 ml einer 6,17 molaren Lösung von HCI in Ether wurde 2 Stunden bei 70° gerührt (nach 7 Minuten war eine Lösung entstanden) und dann im Vakuum eingedampft. Den Rückstand nahm man in 80 ml CH₂Cl₂ auf, schüttelte die Lösung dreimal mit je 15 ml N NaOH und zweimal mit je 7 ml Wasser aus, trocknete die CH₂Cl₂-Lösung über Na₂SO₄ und dampfte nach dem Filtrieren diese Lösung im Vakuum ein. Der verbliebene Rückstand wurde mit 2 ml Ethylacetat angelöst. Nach Zusatz von Ether erfolgte Auskristallisation. Nach 1 Stunde Rühren im Eisbad wurde das Kristallisat abgesaugt, mit Ether nachgewaschen und 15 Stunden bei 100° im Vakuum (5-8 mbar) getrocknet. Man erheilt 1,88 g (= 65,7 % Ausbeute) DC-reines 4-Amino-2-(4-methyl-2-oxoimidazolidin-1-yl)-pyrimidin-5-carbonsäure-(N-ethyl-N-(3-trifluormethyl-phenyl)-amid), Fp. 207-208°.

### Beispiel 1

Eine Suspension von 1,267 g (3 mMol) einer Verbindung II, in der R¹ und R² CH₃, R³, R⁵ und R⁶ Wasserstoff und R⁴ C₂H₅ bedeuten, in 3 ml Eisessig wurde 5 Stunden bei 50° gerührt und anschließend im Vakuum eingedampft. Der verbleibende Rückstand wurde in H₂O/CH₂Cl₂ aufgenommen. Die wäßrige Phase stellte man mit 2 N NaOH auf pH 9. Nach Phasentrennung wurde die wäßrige Phase zweimal mit CH₂Cl₂ extrahiert. Die vereinigten CH₂Cl₂-Extrakte wurden mit H₂O ausgewaschen über MgSO₄ getrocknet, filtriert und im Vakuum eingedampft. Der Rückstand (1,07 g) wurde in Ether gelöst, woraufhin Auskristallisation erfolgte. Die kristalline Masse wurde abgesaugt, mit Ether nachgewaschen und 15 Stunden bei 100° im Vakuum (6 mbar) getrocknet. Man erhielt 0,86 g (67,9 % Ausbeute) DC-reines 4-Amino-2-(4,4-dimethyl-2-oxo-imidazolidin-1-yl)-pyrimidin-5-carbonsäure-(N-ethyl-N-(3-trifluormethyl-phenyl)-amid), Fp. 186 - 187°.

| C₁₉H₂₁F₃N₆O₂ (422,43) | | | | |
|---|---|---|---|---|
| berechnet: | C 54,02 | H 5,01 | F 13,49 | N 19,90 % |
| gefunden: | C 53,5 | H 5,2 | F 12,9 | N 19,7 % |

### Beispiel 2

Zu einer Lösung von 3,80 g (9 mMol) gemäß Beispiel 1 hergestelltem 4-Amino-2-(4,4-dimethyl-2-oxo-imidæolidin-1-yl)-pyrimidin-5-carbonsäure-(N-ethyl-N-(3-trifluormethyl-phenyl)-amid) in 20 ml Aceton tropfte man bei Raumtemperatur 1,52 ml einer 6,6 molaren Lösung von HCI in Ether und rührte die entstandene Suspension 20 Minuten unter Eiskühlung nach. Danach wurde das Kristallisat abgesaugt, mit Aceton und Ether nachgewaschen und 10 Stunden im Vakuum (6-7 mbar) bei 100° getrocknet. Man erhielt 3,83 g (= 92,7 % Ausbeute) 4-Amino-2-(4,4-dimethyl-2-oxo-imidazolidin-1-yl)-pyrimidin-5-carbonsäure-(N-ethyl-N-(3-trifluormethyl-phenyl)-amid)-hydrochlorid, Fp. 264 - 265° (Zers.).

| C₁₉H₂₂ClF₃N₆O₂ (458,89) | | | | | |
|---|---|---|---|---|---|
| berechnet: | C 49,73 | H 4,83 | Cl 7,73 | F 12,42 | N 18,32 % |
| gefunden: | C 48,9 | H 5,0 | Cl 7,6 | F 12,3 | N 17,8 % |

### Beispiel 3

Eine Suspension von 1,746 g (4 mMol) einer Verbindung II, in der R¹ und R² CH₃, R³, R⁵ und R⁶ Wasserstoff und R⁴ Isopropyl bedeuten, in 4 ml Eisessig wurde 3 Stunden bei 50° gerührt und anschließend im Vakuum eingedampft. Der verbleibende Rückstand wurde in 5 ml 2 N Salzsäure und 10 ml Ether gelöst. Nach Trennung der Phasen wurde die salzsäure wäßrige Phase mehrmals mit Ether extrahiert, anschließend mit 6 N Natronlauge auf pH 9-10 gestellt und dann mehrmals mit CH₂Cl₂ extrahiert. Die vereinigten CH₂Cl₂-Extrakte wusch man mit einigen ml Wasser und trocknete sie mit Na₂SO₄. Nach Filtration wurde das CH₂Cl₂ im Vakuum abgezogen und der verbleibende Rückstand in 5-6 ml Ether gelöst. Aus der Etherlösung erfolgte Kristallisation. Die kristalline Substanz wurde abgesaugt, mit Ether gewaschen und 16 Stunden bei 100° getrocknet. Man erhielt 1,14 g (= 65,3 % Ausbeute) DC-reines 4-Amino-2-(4,4-dimethyl-2-oxo-imidazolidin-1-yl)-pyrimidin-5-carbonsäure-(N-isopropyl-N-(3-trifluormethyl-phenyl)-amid), Fp. 202 - 203°.

| C₂₀H₂₃F₃N₆O₂ (436,45) | | | | |
|---|---|---|---|---|
| berechnet: | C 55,04 | H 5,31 | F 13,06 | N 19,26 % |
| gefunden: | C 55,0 | H 5,5 | F 12,4 | N 19,1 % |

### Beispiel 4

Zu einer Lösung von 6,55 g (15 mMol) gemäß Beispiel 3 hergestelltem 4-Amino-2-(4,4-dimethyl-2-oxo-imidazolidin-1-yl)-pyrimidin-5-carbonsäure-(N-isopropyl-N-(3-trifluormethylphenyl)-amid) in 35 ml Aceton tropfte man bei Raumtemperatur 2,5 ml einer 6,6 molaren Lösung von HCI in Ether und rührte die entstandene Suspension 2 Stunden bei Raumtemperatur nach. Dann wurde das Krisallisat abgesaugt, mit Aceton nachgewaschen und 10 Stunden im Vakuum bei 100° getrocknet. Man erhielt 6,84 g (= 96,5 % Ausbeute) 4-Amino-2-(4,4-dimethyl-2-oxo-imidazolidin-1-yl)-pyrimidin-5-carbonsäure-(N-isopropyl-N-(3-trifluormethyl-phenyl)-amid)-hydrochlorid, Fp. 279-280° (Zers.)

| C₂₀H₂₄ClF₃N₆O₂ (472,92) | |
|---|---|
| berechnet: | Cl^{⊖} 7,50 % |
| gefunden: | Cl^{⊖} 7,6 % |

### Beispiel 11

Gemäß der im Beispiel 5 beschriebenen Arbeitsweise wurden 10,0 g (22,3 mMol) einer Verbindung (R¹, R² = CH₃; R³, R⁵, R⁶ = H; R⁴ = Cyclopropylmethyl) und 24,6 ml Eisessig 4 Stunden bei 60° gerührt (nach 20 Minuten Rühren lag eine Lösung vor) und anschließend gemäß Beispiel 5 aufgearbeitet. Man erhielt 6,67 g (66,7 % Ausbeute) DC-reines 4-Amino-2-(4,4-dimethyl-2-oxo-imidazolidin-1-yl)-pyrimidin-5-carbonsäure-(N-cydopropylmethyl-N-(3-trifluormethyl-phenyl)-amid), Fp. 193-194°.

| C₂₁H₂₃F₃N₆O₂ (448,46) | | | | |
|---|---|---|---|---|
| berechnet: | C 56,24 | H 5,17 | F 12,71 | N 18,74 % |
| gefunden: | C 55,9 | H 5,1 | F 12,1 | N 18,9 % |

### Beispiel 12

Gemäß der im Beispiel 6 beschriebenen Arbeitsweise wurden 5,67 g (12,64 mMol) der nach Beispiel 11 hergestellten Verbindung (R¹, R² = CH₃; R³, R⁵, R⁶ =H; R⁴ = Cyclopropylmethyl) in das Hydrochlorid übergeführt. Man erhielt 5,84 g (= 95,3 %) reines 4-Amino-2-(4,4-dimethyl-2-oxo-imidazolidin-1-yl)-pyrimidin-5-carbonsäure-(N-cyclopropylmethyl-N-(3-trifluormethyl-phenyl)-amid)-hydrochlorid, Fp. 265-266°.

| C₂₁H₂₄ClF₃N₆O₂ (484,93) | |
|---|---|
| berechnet: | Cl^{⊖} 7,31 % |
| gefunden: | Cl^{⊖} 7,5 % |

Gemäß der im Beispiel 5 beschriebenen Arbeitsweise wurden die in den Beispielen 13-27 der nachstehenden Tabelle 3 aufgeführten Verbindungen I (R¹, R² = CH₃; R³ = H, vgl. nachstehende Formel) hergestellt.

Von den in der Tabelle 3 aufgeführten Verbindungen (Beispiele 13-27) wurden gemäß der im Beispiel 6 beschriebenen Arbeitsweise die Hydrochloride (Beispiele 28-42) hergestellt, die in der nachstehenden Tabelle 4 zusammengestellt sind.

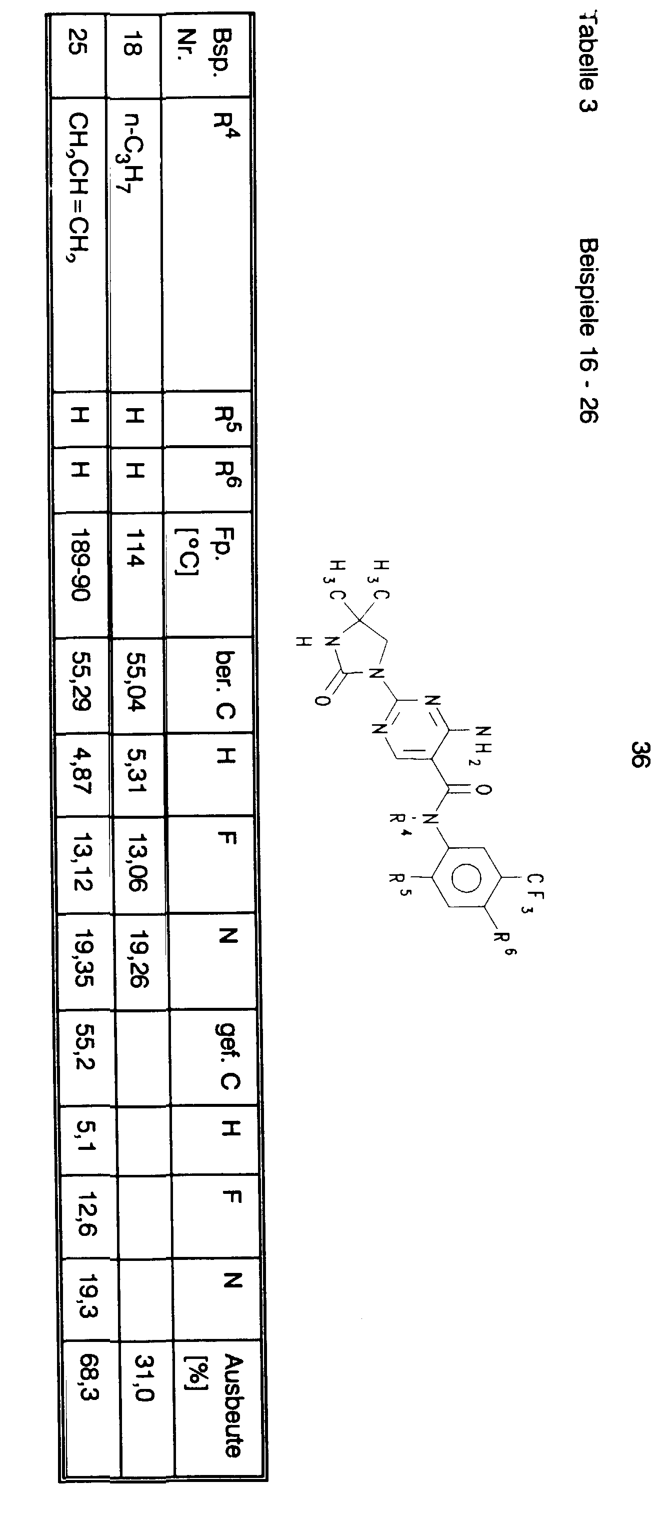

**Tabelle 4**

| Beispiele 31 - 41 | | | | |
|---|---|---|---|---|
| Hydrochloride der in Tabelle 3 in den Beispielen 13 - 27 aufgeführten Verbindungen I | | | | |
| Bsp. Nr. | Hydrochlorid der Verbindung nach Beispiel | Fp. [°C] | ber. Cl | gef. Cl |
| 31 | 16 | 288 | 14,37 | 14,0 |
| 32 | 17 | 278-79 | 7,44 | 7,4 |
| 33 | 18 | 251 | 7,50 | 7,3 |
| 34 | 19 | 293 | 13,98 | 13,7 |
| 35 | 20 | 284-85 | 7,22 | 7,2 |
| 40 | 25 | 268-69 | 7,53 | 7,5 |
| 41 | 26 | 282-83 | 7,05 | 6,6 |

### Beispiel 44

Eine Mischung aus 2,53 g (6 mMol) einer Verbindung II (R¹, R² = CH₃; R³, R⁵, R⁶ = H; R⁴ = C₂H₅), 360 mg (4 mMol) Oxalsäure und 5 ml Propionsäure wurde 2 Stunden bei 70° gerührt (nach 10 Minuten lag eine Lösung vor) und danach im Vakuum eingedampft. Der verbliebene Rückstand wurde wie im Beispiel 43 beschrieben weiter aufgearbeitet. Man erhielt 1,72 g (= 68 % Ausbeute) reines 4-Amino-2-(4,4-dimethyl-2-oxo-imidazolidin-1-yl)-pyrimidin-5-carbonsäure-(N-ethyl-N-(3-trifluormethyl-phenyl)-amid, Fp. 186-187° (vgl. dazu Beispiel 1).

### Beispiel 59

a) Eine Mischung aus 6,34 g (15 mMol) einer Verbindung II (R¹, R² = CH₃; R³, R⁵, R⁶ = H; R⁴ = C₂H₅), 450 mg wasserfreier Oxalsäure und 13 ml Eisessig wurde 3 Stunden bei 65° gerührt und anschließend wie im Beispiel 5 beschrieben aufgearbeitet. Durch Kristallisation aus Ether wurden 4,52 g (= 71,3 % Ausbeute) einer Verbindung I (R¹, R² = CH₃; R³, R⁵, R⁶ = H; R⁴ = C₂H₅), Fp. 186-187° erhalten.
b) In analoger Weise wurde eine Mischung aus 5,07 g (12 mMol) der gleichen Verbindung II, 360 mg wasserfreier Oxalsäure und 10,4 ml wasserfreier Ameisensäure 3 Stunden bei 65° gerührt und anschließend aufgearbeitet. Man erhielt 3,04 g (= 60 % Ausbeute) der Verbindung I (R¹, R² = CH₃; R³, R⁵, R⁶ = H; R⁴ = C₂H₅), Fp. 186-87°.

### Beispiel 60

Eine Suspension von 881 mg (2 mMol) 4-Amino-2-(4,4-dimethyl-2-oxo-imidazolidin-1-yl)-pyrimidin-5-carbonsäure-(N-ethyl-N-(2-fluor-5-trifluormethyl-phenyl)-amid) (Verbindung I nach Beispiel 17) in 13,3 ml Ethylacetat wurde bei Raumtemperatur mit 2,05 ml einer molaren Lösung von HNO₃ in Ethylacetat versetzt. Dabei entstand kurzzeitig eine Lösung, aus der sich eine kristalline Substanz abschied. Diese wurde nach 1 Stunde Rühren (im Eisbad) abgesaugt, mit Ethylacetat und Ether nachgewaschen und 15 Stunden im Vakuum (6-8 mbar) bei 80° getrocknet. Man erhielt 1,02 g 4-Amino-2-(4,4-dimethyl-2-oxo-imidazolidin-1-yl)-pyrimidin-5-carbonsäure-(N-ethyl-N-(2-fluor-5-trifluormethyl-phenyl)-amid-nitrat, Fp. 235-236°. Dieses Nitrat ist in einer Konzentration von 1,7 Gew.-% in Wasser löslich, d.h. 1 Teil löst sich in 57,5 Teilen Wasser.

| C₁₉H₂₁F₄N₇O₅ (503,43) | | | | |
|---|---|---|---|---|
| berechnet: | C 45,33 | H 4,20 | F 15,10 | N 19,48 % |
| gefunden: | C 44,8 | H 4,5 | F 14,6 | N 19,1 % |

### Beispiel 65

Eine Mischung aus 2,535 g (6 mMol) einer Verbindung II (R¹, R² = CH₃; R³, R⁵, R⁶ = H; R⁴ = C₂H₅), 841 mg (4 mMol) Citronensäuremonohydrat und 4,25 ml Eisessig wurde 2 Stunden bei 70° gerührt und anschließend gemäß Beispiel 5 aufgearbeitet. Man erhielt 1,86 g (= 73,4 % Ausbeute) reine Verbindung I (R¹, R² = CH₃; R³, R⁵, R⁶ = H; R⁴ = C₂H₅), Fp. 186-187°.

### Beispiel 67

Mit einer Mischung aus 2,535 g (6 mMol) einer Verbindung II (R¹, R² = CH₃; R³, R⁵, R⁶ = H; R⁴ = C₂H₅), 820 mg (5 mMol) Trichloressigsäure und 4,25 ml Eisessig wurde wie im Beispiel 65 beschrieben verfahren. Man erhielt 1,97 g (77,7 % Ausbeute) DC-reines 4-Amino-2-(4,4-dimethyl-2-oxo-imidazolidin-1-yl)-pyrimidin-5-carbonsäure-(N-ethyl-N-(3-trifluormethyl-phenyl)-amid.

### Beispiel 68

Mit einer Mischung aus 3,04 g (7 mMol) einer Verbindung II (R¹, R² = CH₃; R³, R⁵, R⁶ = H; R⁴ = CH₂CH =CH₂), 665 mg (5,83 mMol) Trifluoressigsäure und 5 ml Eisessig wurde wie im Beispiel 65 beschrieben verfahren. Es wurden 2,00 g (65,8 % Ausbeute) 4-Amino-2-(4,4-dimethyl-2-oxo-imidazolidin-1-yl)-pyrimidin-5-carbonsäure-(N-allyl-N-(3-trifluormethyl-phenyl)-amid), Fp. 187-88° erhalten.

### Beispiel 69

Eine Mischung aus 3,04 g (7 mMol) der gleichen Verbindung II wie im Beispiel 68, 515 mg (4,44 mMol) Fumarsäure und 6 ml Eisessig wurde 1 Stunde bei 85° gerührt und anschließend gemäß Beispiel 5 aufgearbeitet. Man erhielt 2,08 g (= 68,4 % Ausbeute) der gleichen Verbindung I wie im Beispiel 68, Fp. 188-89°.

### Beispiel 78

Eine Mischung aus 3,04 g (7 mMol) einer Verbindung II (R¹, R² = CH₃; R³, R⁵, R⁶ = H; R⁴ = CH₂CH=CH₂), 0,45 ml (∼ 5,8 mMol) Trifluoressigsäure und 5,0 ml Eisessig wurde 2 Stunden bei 70° gerührt und, wie im Beispiel 5 beschrieben aufgearbeitet. Man erhielt 2,00 g (= 65,8 % Ausbeute) der Verbindung I (R¹, R² = CH₃; R³, R⁵, R⁶ = H; R⁴ = CH₂CH=CH₂), Fp. 189°.

### Beispiel 82

Zu einer Suspension von 16,8 g (107,6 mMol) 1-Amidino-4,4-dimethyl-2-oxoimidazolidin (Verbindung III: R¹, R² = CH₃; R³ = H) in 75 ml wasserfreiem Acetonitril tropfte man unter Rühren bei 20° eine Lösung von 40 g (85-90 %igem) 2-Cyano-3-ethoxy-acrylsäure-[N-allyl-N-(3-trifluormethyl-phenyl)-amid] (Verbindung IV: R⁴ = CH₂CH=CH₂; R⁵, R⁶ = H; R⁷ = C₂H₅) in 82 ml wasserfreiem Acetonitril und rührte 2 Stunden bei 20° und 3 Stunden bei 20°-23° nach. Dabei fiel Feststoff aus. Man verdünnte die Mischung mit 150 ml Ether, ließ diese 15 Stunden bei 4-6° stehen, saugte danach den Feststoff ab und wusch ihn mit Ether nach. Der Feststoff wurde in 200 ml Wasser suspendiert, 30 Minuten bei Raumtemperatur gerührt, abgesaugt, mit Wasser nachgewaschen und 48 Stunden bei Raumtemperatur (20°-24°) über P₄O₁₀ im Vakuum (160-180 mbar) getrocknet. Man erhielt 27,8 g (= 59,5 % Ausbeute) 1-Cyano-1-[N-allyl-N-(3-trifluormethylphenyl)-carbamoyl]-2-[(imino-(4,4-dimethyl-2-oxo-imidazolidin-1-yl)-methyl)-amino]-ethen (Verbindung II: R¹, R² = CH₃; R³, R⁵, R⁶ = H; R⁴ = CH₂CH=CH₂), Fp. 184-185°.

| C₂₀H₂₁F₃N₆O₂ (434,44) | | | | |
|---|---|---|---|---|
| berechnet: | C 55,29 | H 4,87 | F 13,12 | N 19,35 % |
| gefunden: | C 54,7 | H 4,6 | F 12,5 | N 18,9 % |

### Beispiel 85

Zu einer Suspension von 11,0 g (70 mMol) 1-Amidino-4,4-dimethyl-2-oxo-imidazolidin in 45 ml abs. Dimethoxyethan (DME) tropfte man unter Rühren bei 18°-20° eine Lösung von 27,5 g (80-85 %igem) 2-Cyano-3-ethoxy-acrylsäure-[N-cyclopropylmethyl-N- (3-trifluormethyl-phenyl)-amid (Verbindung IV: R⁴ = CH₂- ◁, R⁵, R⁶ = H; R⁷ = C₂H₅) in 48 ml abs. DME und rührte 3,5 Stunden bei 20-23° nach. Danach dampfte man die Reaktionsmischung im Vakuum bei maximal 25° Badtemperatur ein. Der ölige Rückstand wurde in 200 ml Ether gelöst und mit 50 ml 2 N HCI durchgeschüttelt. Dabei fiel Kristallisat (Hydrochlorid der gebildeten Verbindung II (R¹, R² = CH₃; R³, R⁵, R⁶ = H; R⁴ = CH₂-◁)) aus. Dieses wurde abgesaugt und mit Ether und mit Wasser nachgewaschen. Danach suspendierte man diesen kristallinen Stoff in 150 ml Wasser und tropfte bei 10-18° 2 N NaOH zu, bis sich ein pH-Wert von 8,7-8,9 einstellte. Man rührte 1 Stunde unter Eiskühlung nach, saugte den Feststoff ab, wusch diesen mit Wasser nach und trocknete ihn 50 Stunden im Vakuum (6-8 mbar) über P₂O₅ bei 22-24°. Man erhielt 11,2 g (38,3 % Ausbeute)1-Cyano-1-[N-cyclopropylmethyl-N-(3-trifluormethyl-phenyl)-carbamoyl]-2-[(imino-(4,4-dimethyl-2-oxo-imidazolidin-1-yl)-methyl)-amino]-ethen (Verbindung II: R¹, R² = CH₃; R³, R⁵, R⁶ = H; R⁴ = CH₂-◁), Fp. 180-181° (Zers.).

| C₂₁H₂₃F₃N₆O₂ (448,46) | | | | |
|---|---|---|---|---|
| berechnet: | C 56,24 | H 5,17 | F 12,71 | N 18,74 % |
| gefunden: | C 56,4 | H 5,1 | F 12,4 | N 19,1 % |

### Beispiel 86

Eine Suspension von 449 mg (1 mMol) der gemäß Beispiel 85 erhaltenen Verbindung II in 2,5 ml Wasser wurde bei Raumtemperatur mit 0,5 ml 2 N HCI versetzt, 10 Minuten bei Raumtemperatur und 40 Minuten unter Eiskühlung gerührt und dann abgesaugt. Das Kristallisat wurde 28 Stunden bei 23-25° im Vakuum (6-8 mbar) über P₄O₁₀ getrocknet. Erhalten wurden 0,46 g (94,9 % Ausbeute) 1-Cyano-1-[N-cyclopropylmethyl-N-(3-trifluormethyl-phenyl)-carbamoyl]-2-[(imino-(4,4-dimethyl-2-oxo-imidazolidin-1-yl)-methyl)-amino]-ethen-hydrochlorid, Fp. 179-180°.

| C₂₁H₂₄ClF₃N₆O₂ (484,93) | |
|---|---|
| berechnet: | Cl^{⊖} 7,31 % |
| gefunden: | Cl^{⊖} 7,4 % |

### Beispiel 87

Zu einer Suspension von 3,91 g (25 mMol) 1-Amidino-4,4-dimethyl-2-oxoimidazolidin in 16 ml abs. DME tropfte man bei 20° eine Lösung von 8,2 g (nicht gereinigt, ca. 90 %ig) 2-Cyano-3-ethoxy-acrylsäure-[N-isopropyl-N-(3-trifluormethylphenyl)-amid (Verbindung IV: R⁴ = CH(CH₃)₂; R⁵, R⁶ = H; R⁷ = C₂H₅) in 20 ml abs. DME und rührte 4,5 Stunden bei 22-24° nach. Danach dampfte man die Mischung im Vakuum bei maximal 25° Badtemperatur ein. Der ölige Rückstand wurde in 250 ml Ether aufgenommen und sechsmal mit je 25 ml Wasser ausgeschüttelt. Danach wurde die Etherlösung über MgSO₄ getrocknet, filtriert und im Vakuum (< 26°) eingedampft. Der Rückstand (12,7 g) war teils kristallin. Man nahm diesen in 40 ml Ether auf, gab 2 ml Pentan zu, ließ 2 Tage bei 4-6° stehen und saugte anschließend das Kristallisat ab. Nach dem Trocknen (48 Stunden, 6-8 mbar, 20-24° über P₄O₁₀) wurden 5,12 g (= 47 % Ausbeute) DC-reine Verbindung II (R¹, R² = CH₃; R³, R⁵, R⁶ = H; R⁴ = CH(CH₃)₂), Fp. 121-122°, erhalten.

| C₂₀H₂₃F₃N₆O₂ (436,45) | | | | |
|---|---|---|---|---|
| berechnet: | C 55,04 | H 5,31 | F 13,06 | N 19,26 % |
| gefunden: | C 54,9 | H 5,6 | F 12,4 | N 18,5 % |

### Beispiel 88

In der gleichen Weise wie im Beispiel 86 beschrieben wurde 1 mMol (437 mg) der gemäß Beispiel 87 hergestellten Verbindung II in das Hydrochlorid übergeführt. Erhalten wurden 400 mg (84,6 % Ausbeute) 1-Cyano-1-[N-isopropyl-N-(3-trifluormethyl-phenyl)-carbamoyl]-2-[(imino-(4,4-dimethyl-2-oxo-imidazolidin-1-yl)-methyl)-amino]-ethen-hydrochlorid, Fp. 258-260°.

| C₂₀H₂₄ClF₃N₆O₂ (472,92) | |
|---|---|
| berechnet: | Cl^{⊖} 7,50 % |
| gefunden: | Cl^{⊖} 7,4 % |

### Beispiel 89

In der gleichen Weise wie im Beispiel 86 beschrieben, wurde 1 mMol (479 mg) der gemäß Beispiel 97 hergestellten Verbindung II in das Hydrochlorid übergeführt. Erhalten wurden 1,02 g (99 %) 1-Cyano-1-[N-(1,3-dimethyl-1-butyl)-N-(3-trifluormethyl-phenyl)-carbamoyl]-2-[(imino-(4,4-dimethyl-2-oxo-imidazolidin-1-yl)-methyl)-amino]-ethen-hydrochlorid, Fp. 255-256°.

| C₂₃H₃₀ClF₃N₆O₂ (515,00) | | | | | |
|---|---|---|---|---|---|
| berechnet: | C 53,64 | H 5,87 | Cl^{⊖} 6,88 | F 11,07 | N 16,32 % |
| gefunden: | C 53,4 | H 5,9 | Cl^{⊖} 6,4 | F 10,6 | N 16,1 % |

Gemäß den in den Beispielen 82 und 84, 85 und 87 beschriebenen Arbeitsweisen, die sich vor allem im Vorgehen bei der Aufarbeitung unterscheiden, wurden die in der folgenden Tabelle 5 aufgeführten Verbindungen II (Beispiele 90-112) hergestellt. Bei den einzelnen Verbindungen der Tabelle 5 ist jeweils auf das Beispiel, demgemäß diejenige Verbindung II hergestellt wurde, verwiesen.

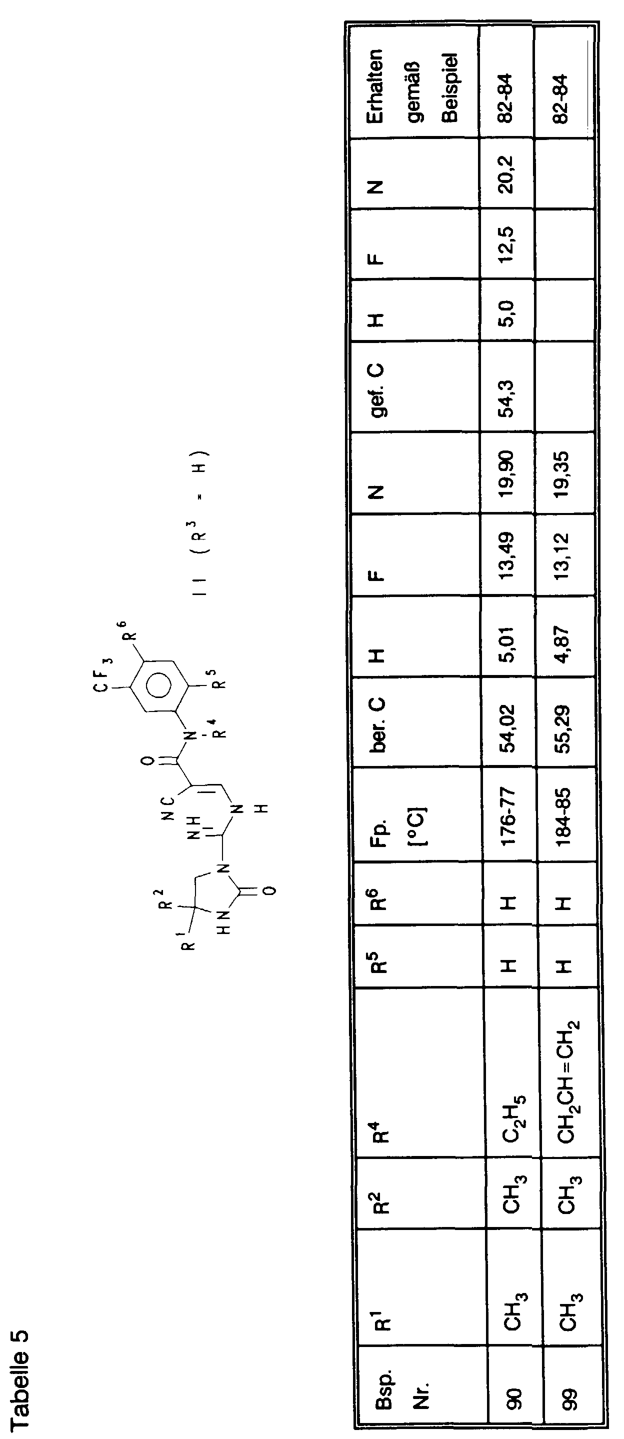

Gemäß Beispiel 113 bzw. Beispiel 6 wurden die in der folgenden Tabelle 6 (Beispiele 114 bis 117) aufgeführten Hydrochloride von Verbindungen I hergestellt.

**Tabelle 6**

| Beispiele 114 - 117 | | | | |
|---|---|---|---|---|
| Beispiel Nr. | Hydrochlorid der Verbindung I nach Beispiel | Fp. [°C] | berechnet Cl⁻% | gefunden Cl⁻% |
| 114 | 74-76 | 276-77 | 7,50 | 7,7 |
| 115 | 81 | 284-85 | 6,83 | 6,8 |
| 116 | 80 | 269-70 | 7,31 | 7,5 |
| 117 | 77 | 247-48 | 7,11 | 7,2 |

### Beispiel 123

Herstellung einer Filmtablette nachfolgend genannter Zusammensetzung:

| Bestandteile (pro Tablette) | Gewicht (mg) |
|---|---|
| 1. Verbindung I gemäß Beispiel 2 | 100 |
| 2. Lactose | 35 |
| 3. Maisstärke | 20 |
| 4. Mikrokristalline Cellulose | 10 |
| 5. Natrium-Stärkeglykolat | 8 |
| 6. Siliziumdioxid | 5 |
| 7. Magnesiumstearat | 2 |
| 8. Filmlack | 10 |
| Gesamtgewicht | 190 |

a) Die Substanzen 1. - 4. werden gemischt, kompaktiert und durch ein Frewitt-Sieb (Maschenweite 1,0 mm) passiert.
b) Das Granulat aus a) wird mit den Substanzen 5. - 7. bestäubt und verpreßt.
c) Die Tabletten aus b) werden mit wäßrigem Filmlack lackiert.

### Beispiel 124

Herstellung einer Filmtablette nachfolgend genannter Zusammensetzung

| Bestandteile (pro Tablette) | Gewicht (mg) |
|---|---|
| 1. Verbindung I gemäß Beispiel 12 | 100 |
| 2. Lactose | 11 |
| 3. Hydroxypropylmethylcellulose | 60 |
| 4. Mikrokristalline Cellulose | 7 |
| 5. Magnesiumstearat | 2 |
| 6. Filmlack | 10 |
| Gesamtgewicht | 190 |

a) Die Substanzen 1. - 4. werden gemischt, kompaktiert und durch ein Frewitt-Sieb (Maschenweite 1,0 mm) passiert.
b) Das Granulat aus a) wird mit der Substanz 5. bestäubt und verpreßt.
c) Die Tabletten aus b) werden mit wäßrigem Filmlack lackiert.

Bei dieser Tablettenart handelt es sich um eine Zubereitung mit verzögerter Wirkstoff-Freigabe.

## Patentansprüche

1. 4-Amino-2-ureido-pyrimidin-5- carbonsäure-amide der Formel I, 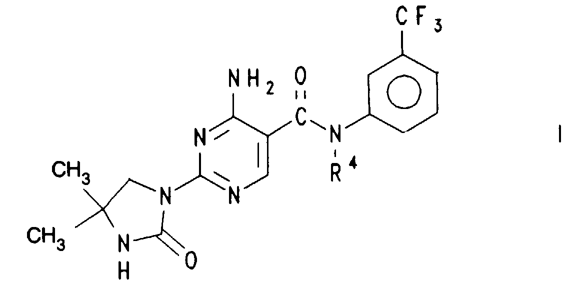 in der
R⁴ (C₂-C₃)-Alkyl, Allyl oder Cyclopropylmethyl
bedeuten,
sowie deren physiologisch verträgliche Säureadditionssalze.

2. Verfahren zur Herstellung von 4-Amino-2-ureido-pyrimidin-5-carbonsäure-amiden der Formel I gemäß Anspruch 1 und von deren gut wasserlöslichen Salzen, dadurch gekennzeichnet, daß man eine Verbindung der Formel II, 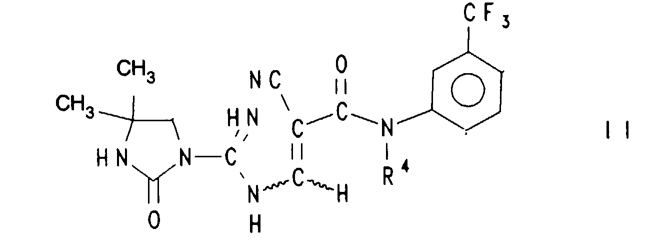 in der
R⁴ und die zu Formel I angegebene Bedeutung hat, oder ein Salz oder ein Salzgemisch einer Verbindung II bei einer Temperatur von 0° bis 240°C mit oder ohne Lösemittelzusatz zu einer Verbindung der Formel I oder einem Salz oder einem Salzgemisch einer Verbindung I cyclisiert, eine erhaltene Verbindung der Formel I gegebenenfalls in ein in Wasser gut lösliches Salz überführt oder ein erhaltenes Salz oder Salzgemisch gegebenenfalls in ein in Wasser gut lösliches Salz überführt.

3. Verbindungen der Formel II 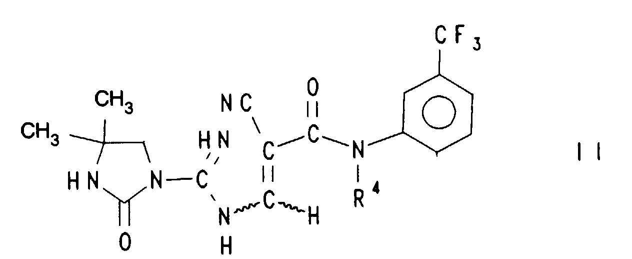 in der die zu Formel I des Anspruchs 1 angegebene Bedeutung hat sowie deren Säureadditionssalze.

4. Verfahren zur Herstellung der Verbindungen der Formel II und von deren Salzen, dadurch gekennzeichnet, daß man eine Verbindung der Formel III, 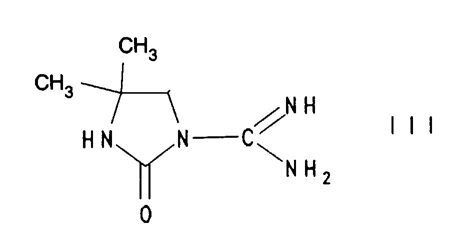 oder ein Salz dieser Verbindung III mit einer Verbindung der Formel IV, 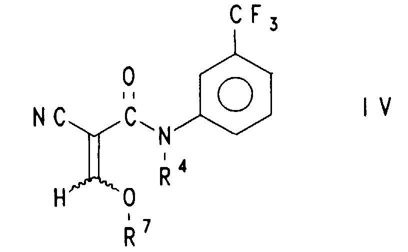 in der R⁴, die zu Formel II angegebene Bedeutung hat und R⁷ (C₁-C₄)-Alkyl bedeutet, in Gegenwart eines organischen Löse- oder Verdünnungsmittels unter Bildung einer Verbindung der Formel II oder einem Salz dieser Verbindung umsetzt, und eine erhaltene Verbindung der Formel II gegebenenfalls in ein Salz überführt oder ein erhaltenes Salz gegebenenfalls in eine Verbindung der Formel II überführt.

5. Arzneimittel enthaltend eine Verbindung gemäß Anspruch 1.

6. Verwendung einer Verbindung gemäß Anspruch 1 als Arzneimittel.

7. Verwendung einer Verbindung gemäß Anspruch 1 zur Herstellung eines Arzneimittels zur Behandlung von Lipidstoffwechselstörungen, die durch eine Stimulierung des hepatischen LDL-Rezeptors günstig beeinflußt werden.

8. Verwendung einer Verbindung gemäß Anspruch 1 zur Stimulierung des hepatischen LDL-Rezeptors.

## Claims

1. A 4-amino-2-ureidopyrimidine-5-carboxamide of the formula I 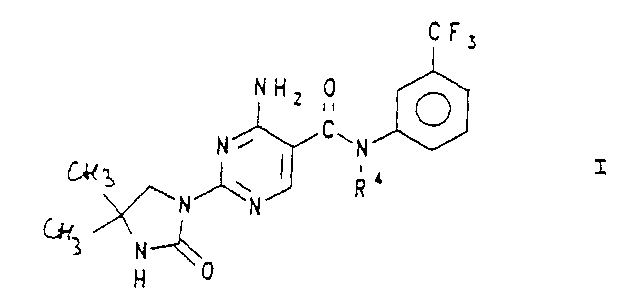 in which
R⁴ is (C₂-C₃)-alkyl, allyl or cyclopropylmethyl, and the physiologically tolerated acid addition salts thereof.

2. A process for the preparation of 4-amino-2-ureidopyrimidine-5-carboxamides of the formula I as claimed in claim 1 and of the salts thereof which are readily soluble in water, which comprises cyclizing a compound of the formula II 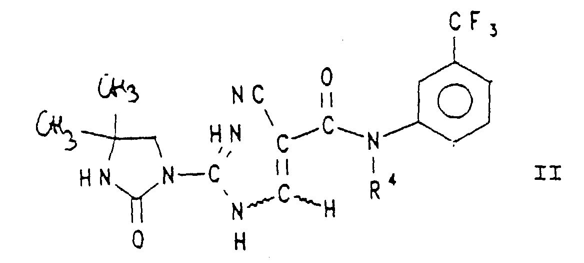 in which
R⁴ has the meaning indicated for formula I, or a salt or a mixture of salts of a compound II at a temperature from 0° to 240°C with or without added solvent to give a compound of the formula I or a salt or a mixture of salts of a compound I, where appropriate converting a resulting compound of the formula I into a salt which is readily soluble in water or where appropriate converting a resulting salt or mixture of salts into a salt which is readily soluble in water.

3. A compound of the formula II 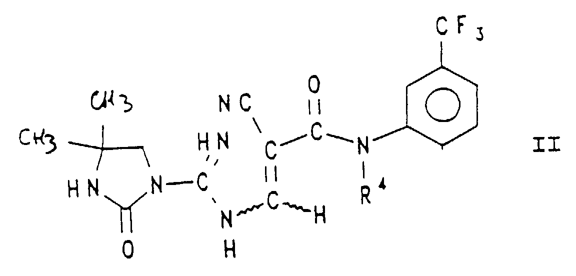 in which R⁴ has the meaning indicated for formula I of claim 1,
and the acid addition salts thereof.

4. A process for the preparation of the compounds of the formula II and of the salts thereof, which comprises reacting a compound of the formula III 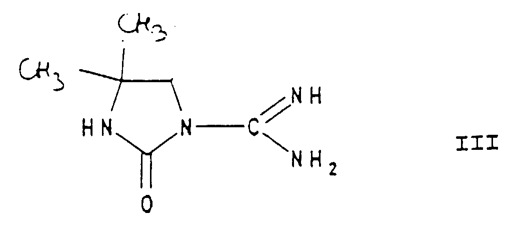 or a salt of this compound III, with a compound of the formula IV 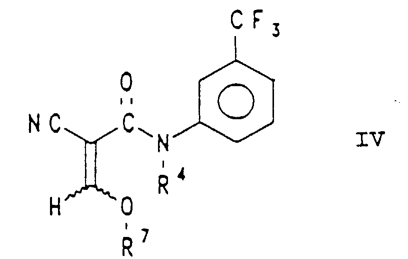 in which R⁴ has the meaning indicated for formula II, and R⁷ is (C₁-C₄)-alkyl, in the presence of an organic solvent or diluent to form a compound of the formula II or a salt of this compound, and where appropriate converting a resulting compound of the formula II into a salt, or where appropriate converting a resulting salt into a compound of the formula II.

5. A pharmaceutical containing a compound as claimed in claim 1.

6. The use of a compound as claimed in claim 1 as pharmaceutical.

7. The use of a compound as claimed in claim 1 for the production of a pharmaceutical for the treatment of lipid metabolism disorders which are beneficially influenced by a stimulation of the hepatic LDL receptor.

8. The use of a compound as claimed in claim 1 for stimulation of the hepatic LDL receptor.

## Revendications

1. 4-amino-2-uréido-pyrimidine 5-carboxamides de formule I, 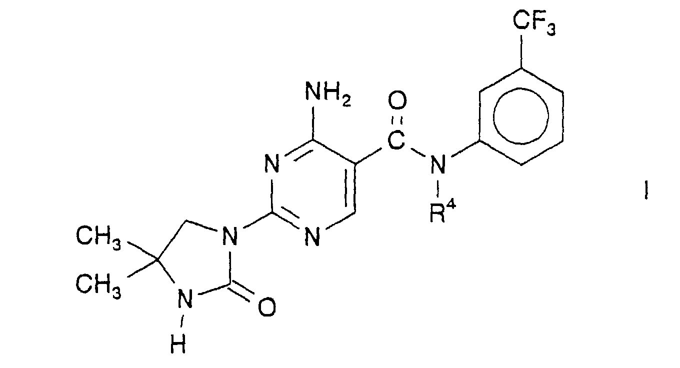 dans laquelle
R⁴ représente un groupe alkyle (C₂-C₃), allyle ou cyclopropylméthyle, ainsi que leurs sels obtenus par addition d'acide physiologiquement acceptables.

2. Procédé de préparation de 4-amino-2-uréido-pyrimidine 5-carboxamides de formule I selon la revendication 1 et de leurs sels facilement solubles dans l'eau, caractérisé en ce que l'on cyclise un composé de formule II, 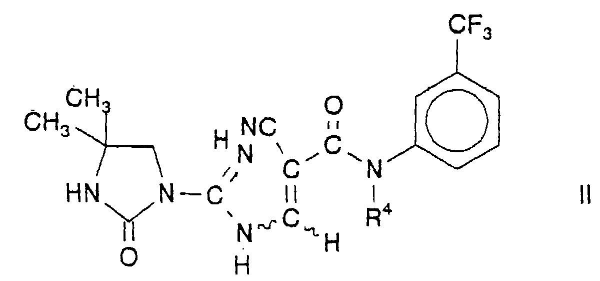 dans laquelle R⁴ a la signification donnée pour la formule I,
ou un sel ou un mélange de sels d'un composé Il, à une température de 0 à 240 °C, avec ou sans ajout de solvant, pour former un composé de formule I ou un sel ou un mélange de sels d'un composé I, on transforme éventuellement un composé de formule I obtenu en un sel facilement soluble dans l'eau ou on transforme éventuellement un sel ou un mélange de sels obtenu en un sel facilement soluble dans l'eau.

3. Composés de formule Il 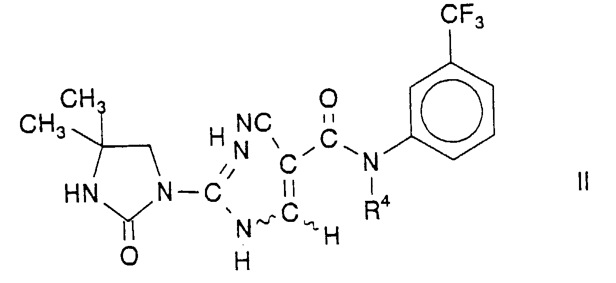 dans laquelle R⁴ a la signification donnée pour la formule I dans la revendication 1, ainsi que leurs sels obtenus par addition d'acide.

4. Procédé de préparation des composés de formule Il et de leurs sels, caractérisé en ce que l'on fait réagir un composé de formule III 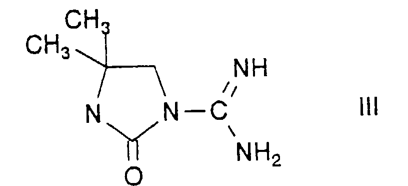 ou un sel de ce composé III, avec un composé de formule IV, 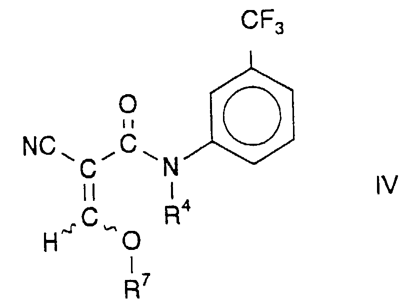 dans laquelle R⁴ a la signification donnée pour la formule Il et R⁷ représente un groupe alkyle (C₁-C₄), en présence d'un solvant ou d'un diluant organique pour former un composé de formule Il ou un sel de ce composé, et que l'on transforme éventuellement un composé de formule II obtenu en un sel ou que l'on transforme éventuellement un sel obtenu en un composé de formule II.

5. Médicament contenant un composé selon la revendication 1.

6. Utilisation d'un composé selon la revendication 1 comme médicament.

7. Utilisation d'un composé selon la revendication 1 pour la préparation d'un médicament pour le traitement de disfonctionnements du métabolisme lipidique influencé favorablement par une stimulation du récepteur hépatique LDL.

8. Utilisation d'un composé selon la revendication 1 pour la stimulation du récepteur hépatique LDL.
